# EUROPEAN PATENT APPLICATION

(11) **EP 4 079 325 A1**
(43) Date of publication of application: **26.10.2022**
(21) Application number: 20902019.7
(22) Date of filing: 19.11.2020
(51) Int. Cl.: A61K 47/10, A61K 9/10, A61K 9/19, A61K 31/496, A61K 47/26, A61K 47/34, A61M 5/28, A61P 25/18

(54) **AGGREGATION INHIBITORY AGENT AND MEDICAL COMPOSITION AND MEDICAL DEVICE INCLUDING SAME**

(30) Priority: 16.12.2019 JP 2019226679
(71) Applicant: Nipro Corporation, Osaka-shi, Osaka 531-8510 (JP)
(72) Inventor: FUJITA, Mizuno, Osaka-shi, Osaka 531-8510 (JP); MORITANI, Miyuki, Osaka-shi, Osaka 531-8510 (JP); SATO, Shinkichi, Osaka-shi, Osaka 531-8510 (JP); HATSUSHIKA, Minoru, Osaka-shi, Osaka 531-8510 (JP)
(74) Representative: Gerauer, Marc Philippé
(86) International application number: PCT/JP2020/043297
(87) International publication number: WO 2021/124793

(57) **Abstract**

An anti-aggregation agent of the present invention contains, as an anti-aggregation ingredient, at least one selected from the group consisting of polyoxyethylene cetyl ether, polyoxyethylene sorbitan fatty acid esters, and polyoxyethylene polyoxypropylene glycols having an average molecular weight of 3000 to 13000. According to the present invention, in a container whose inner wall is treated with silicone oil, aggregation of a poorly water-soluble drug caused by the silicone oil can be prevented.

## Description

### Technical Field

The present invention relates an anti-aggregation agent, as well as a pharmaceutical composition and a medical device using the same.

### Background Art

To administer a poorly water-soluble drug, such as aripiprazole hydrate used for the treatment of schizophrenia, as an injection preparation, the drug is dispersed in water into the form of an aqueous suspension beforehand, and the aqueous suspension is directly injected into a patient, or the drug is prepared as a solid (lyophilized product or the like) in the form of powder or cake, the solid is suspended in water at the time of use, and the suspension is injected into the patient. Here, the aqueous suspension or the solid (lyophilized product or the like) is stored and distributed in a state in which it is accommodated in a vial or a so-called prefilled syringe such as a double-chamber syringe. In the case where the aqueous suspension or the solid is accommodated in a vial, it is extracted therefrom using a general-purpose syringe at the time of use and administered to the patient.

In the prefilled syringe and the general-purpose syringe above, in order to improve slidability inside the syringe, silicone treatment is applied to an inner wall of the syringe (specifically, silicone oil, such as a silicone oil or a silicone oil derivative, is applied thereto). Moreover, silicone treatment may also be applied to an inner wall of the vial for the purpose of improving water repellency or for other purposes.

However, when silicone oil is present on the inner wall of a syringe (a prefilled syringe or a general-purpose syringe) or the inner wall of a vial as described above, the aqueous suspension or the lyophilized product accommodated in the syringe or the vial may be contaminated with the silicone oil. It has been pointed out that, if contamination with silicone oil occurs, when the contaminated aqueous suspension or lyophilized product is resuspended in an aqueous dispersion medium, the poorly water-soluble drug aggregates in the resuspension liquid, leading to an increase in the particle size of the drug that is present in the resuspension liquid. The drug with the thus increased particle size exhibits a dissolution profile different from that of the drug itself (i.e., the drug before being contaminated with silicone oil), and this affects the efficacy of the poorly water-soluble drug, for example, causing a change in the sustained-release properties thereof, and can result in clogging in the device when the drug is used as an injection preparation, physical irritation to an injection site of the patient due to the increased particle size, and the like.

To address this issue, Patent Document 1 proposes the suppression of aggregation of aripiprazole caused by a silicone oil, by improving the shape of a medicinal cake accommodated in the above-described prefilled syringe (medical device) and thereby preventing the cake from coming into contact with an inner surface of the syringe. Also, Patent Document 2 proposes the suppression of aggregation of aripiprazole caused by a silicone oil, by adjusting the amount of the silicone oil with respect to that of aripiprazole used to a predetermined range. Both of these technologies attempt to suppress aggregation of aripiprazole by reducing the amount of contamination with silicone oil.

However, even with the above-described technologies, contamination with silicone oil cannot be completely avoided, and it is still difficult to say that aggregation of a poorly water-soluble drug when suspended can be sufficiently suppressed. Thus, there is demand for further technological development for preventing a poorly water-soluble drug from aggregating in a suspension.

### Related Art Documents

### Patent Documents

Patent Document 1: Japanese Patent No. 5980215
Patent Document 2: Japanese Patent No. 5575269

### Summary of the Invention

### Problem to be Solved by the Invention

The present invention was made in order to solve the aforementioned problems, and it is an object thereof to provide an anti-aggregation agent that enables a poorly water-soluble drug stored in the presence of silicone oil to be appropriately suspended in an aqueous dispersion medium and that can prevent aggregation of the drug, as well as a pharmaceutical composition and a medical device using the anti-aggregation agent.

### Means for Solving the Problem

The present invention is an anti-aggregation agent for preventing aggregation of a poorly water-soluble drug caused by silicone oil,
comprising, as an anti-aggregation ingredient, at least one selected from the group consisting of polyoxyethylene cetyl ether, polyoxyethylene sorbitan fatty acid esters, and polyoxyethylene polyoxypropylene glycols having an average molecular weight of 3000 to 13000.

The present invention is also a pharmaceutical composition comprising a poorly water-soluble drug and the anti-aggregation agent in the presence of silicone oil.

In one embodiment of the pharmaceutical composition of the present invention, the anti-aggregation agent contains the anti-aggregation ingredient in an amount of 2 to 40 parts by mass with respect to 100 parts by mass of the drug.

In one embodiment of the pharma composition of the present invention, the silicone oil is derived from an inner wall of a container, and the inner wall being treated with silicone oil.

In one embodiment, the pharmaceutical composition of the present invention is obtained through lyophilization.

The present inventio is also a medical device comprising a container whose inner wall is treated with silicone oil,
wherein a poorly water-soluble drug and the anti-aggregation agent according to claim 1 are accommodated in the container.

In one embodiment, the medical device of the present invention has a form of a prefilled syringe.

In one embodiment of the medical device of the present invention, the anti-aggregation agent contains the anti-aggregation ingredient in an amount of 2 to 40 parts by mass with respect to 100 parts by mass of the drug.

### Effects of the Invention

According to the present invention, even if silicone oils are applied to the inner wall of a container constituting a medical device, the poorly water-soluble agent stored in the container aggregates when suspended in an aqueous dispersion medium. Therefore, it is possible to prevent the particle size from increasing. As a result, the dissolution profile of the poorly water-soluble drug itself to be used does not deviate significantly from the dissolution profile of the drug when it is not in contact with silicone oils, and substantially the same sustained release property and drug effect can be obtained.

### Description of Embodiments

Hereinafter, the present invention will be described in detail.

### (1) Anti-Aggregation Agent

An anti-aggregation agent of the present invention is a composition used to prevent aggregation of a poorly water-soluble drug caused by silicone oil.

### (Silicone Oil)

The term "silicone oil" as used herein encompasses silicone oils and silicone oil derivatives that can be used in pharmaceutical applications, as well as combinations thereof. A more specific example of the silicone oils is dimethylpolysiloxane. More specific examples of the silicone oil derivatives include those in which a portion of a side chain and/or an end of silicone is substituted with a polyoxyalkylene group or a vinyl group, for example.

The average molecular weight of silicone constituting silicone oil is, without limitation, preferably 10 to 100,000,000, more preferably 100 to 10,000,000, and even more preferably 200 to 10,000.

### (Poorly Water-Soluble Drug)

The term "poorly water-soluble drug" as used herein encompasses drugs that are classified as "sparingly soluble", "slightly soluble", "very slightly soluble", or "practically insoluble" as defined in the Japanese Pharmacopoeia. More specifically, the term "poorly water-soluble drug" encompasses drugs with respect to which, for example, when 1 g or 1 mL of a solute (drug) is added to a solvent (water) and the mixture at 20±5°C is shaken vigorously for 30 seconds every 5 minutes, the amount of solvent (water) required for dissolving the solute within 30 minutes is from 30 mL to less than 100 mL, from 100 mL to less than 1000 mL, from 1000 mL to less than 10000 mL, and 10000 mL or more.

Examples of the poorly water-soluble drug include, but are not limited to, aripiprazole, candesartan, ezetimibe, atorvastatin, tolvaptan, sorafenib, paclitaxel, adriamycin, camptothecin, cisplastin, daunomycin, Pinorubin, methotrexate, mitomycin C, etoposide, gefitinib, irinotecan hydrochloride, topotecan hydrochloride, docetaxol, vinblastine sulfate, vincristine sulfate, vindesine sulfate, teniposide, vinorelbine tartrate, busulfan, carboquone, thiotepa, cyclophosphamide, melphalan, estramustine phosphate sodium, mechlorethamine oxide hydrochloride, ifosfamide, ranimustine, nimustine hydrochloride, bleomycin hydrochloride, peplomycin sulfate, zinostatin stimalate, actinomycin D, aclarubicin hydrochloride, doxorubicin hydrochloride, idarubicin hydrochloride, amrubicin hydrochloride, daunorubicin hydrochloride, pirarubicin, epirubicin hydrochloride, valrubicin, mercaptopurine, fludarabine phosphate, cladribine, fluorouracil, tegafur, cytarabine, gemcitabine hydrochloride, cytarabine ocfosphate, capecitabine, lentinan, oxaliplatin, doxifluridine, carmofur, enocitabine, nedaplatin, carboplatin, fadrozole hydrochloride, anastrozole, exemestane, bicalutamide, flutamide, tamoxifen citrate, toremiphene citrate, tretinoin, pentostatin, L-asparaginase, dacarbazine, procarbazine hydrochloride, mitoxantrone hydrochloride, sobuzoxane, trastuzumab, rituximab, imatinib mesylate, 5-fluoro-2'-deoxyuridine, Ascre, Carbocrin, Kinolespan, Krestin, Picibanil, hydrocortisone, hydrocortisone acetate, prednisolone, methylprednisolone, prednisolone acetate, hydrocortisone acetate propionate, prednisolone valerate, dexamethasone, betamethasone, triamcinolone, clobetasone acetate, clobetasol propionate, fluocinonide, dexamethasone acetate, betamethasone valerate, triamcinolone acetonide, aspirin, salicylic acid, acetaminophen, methyl salicylate, glycol salicylate, mefenamic acid, flufenamic acid, indomethacin, diclofenac, ketoprofen, ibuprofen, flurbiprofen, fenprofen, bufexamac, piroxicam, oxyphenbutazone, mepirizole, ibuprofen piconol, clidanac, phenylbutazone, naproxen, glycyrrhizin, glycyrrhetinic acid, azulene, camphor, thymol, 1-menthol, tolfenamic acid, sasapyrine, alclofenac, diclofenac, suprofen, loxoprofen, acemetacin, metiazinic acid, protizinic acid, sulindac, pranoprofen, fentiazac, diflunisal, tiaprofenic acid, oxaprozin, felbinac, bufexamac, dibucaine hydrochloride, ethyl aminobenzoate, procaine hydrochloride, lidocaine, tetracaine hydrochloride, lidocaine hydrochloride, teecaine, benzyl alcohol, pramoxine hydrochloride, quatacaine hydrochloride, butanicaine hydrochloride, piperocaine hydrochloride, chlorobutanol, barbital, amobarbital, amobarbital sodium, phenobarbital, phenobarbital sodium, secobarbital sodium, pentobarbital calcium, hexobarbital, triclofos, bromovalerylurea, glutethimide, methaqualone, perlapine, nitrazepam, estazolam, flurazepam hydrochloride, flunitrazepam, estazolam, chloramphenicol, cefmetazole, bacitracin, penicillin, cephalexin, tetracycline, streptomycin, nystatin, erythromycin, fradiomycin sulfate, tolnaftate, tocopherol acetate, benzyl nicotinate, tolazoline, verapamil, caffeine, cyclandelate, acetylcholine, tocopherol nicotinate, nifedipine, dipyridamole, prenylamine lactate, efloxate, phenytoin, phenacemide, ethylphenacemide, ethotoin, primidone, phensuximide, nitrazepam, chronazepam, carbamazepine, clozobazone, phenopropamate, diphenhydramine, mequitazine, digoxin, digotoxin, ubidecarenone, phenytoin, disopyramide, polythiazide, spironolactone, chlorthalidone, deserpidine, meptame, reserbine, mebutamate, prostaglandin F2a danazol, mepitiostane, and pharmaceutically acceptable salts, hydrates, and derivatives thereof.

### (Anti-Aggregation Ingredient)

The anti-aggregation agent of the present invention contains an anti-aggregation ingredient. Examples of the anti-aggregation ingredient include polyoxyethylene cetyl ether, polyoxyethylene sorbitan fatty acid esters, and polyoxyethylene polyoxypropylene glycols having an average molecular weight of 3000 to 13000, as well as combinations thereof.

Polyoxyethylene sorbitan fatty acid esters are obtained by the ester reaction of polyoxyethylene sorbitan with long-chain fatty acids (e.g., lauric acid, stearic acid, palmitic acid, oleic acid, and the like), and are also called polysorbates. Specific examples of the polyoxyethylene sorbitan fatty acid esters include polysorbate 20 (Tween 20), polysorbate 40 (Tween 40), polysorbate 60 (Tween 60), polysorbate 80 (Tween 80), polysorbate 85 (Tween 85), and polysorbate 120, as well as combinations thereof.

The polyoxyethylene polyoxypropylene glycols having an average molecular weight of 3000 to 13000 are Pluronic (registered trademark) type polyoxyethylene polyoxypropylene glycols, and preferably polyoxyethylene polyoxypropylene glycols having an average molecular weight of 5500 to 12500. Specific examples of such polyoxyethylene polyoxypropylene glycols include polyoxyethylene (160) polyoxypropylene (30) glycol and the like.

Note that, as used herein, the "average molecular weight" of polyoxyethylene polyoxypropylene glycols means the weight average molecular weight, and is measured by GPC (gel permeation chromatography), for example.

### (Other Ingredients)

The anti-aggregation agent of the present invention is preferably composed only of the above-described anti-aggregation ingredient, but may also contain other ingredients, in addition to the anti-aggregation ingredient, as long as the effects of the present invention are not impaired. There is no particular limitation on the other ingredients, and known additives can be used. When the anti-aggregation agent contains any other ingredient, the content of the anti-aggregation ingredient in the anti-aggregation agent of the present invention is preferably 50 % by mass or greater, more preferably 80 % by mass or greater, and even more preferably 90 % by mass or greater.

The anti-aggregation agent of the present invention is prepared, for example, in the form of a pharmaceutical composition in which it is mixed with a poorly water-soluble drug, then is accommodated in a container whose inner wall is treated with silicone oil, and prevents the drug from aggregating in the presence of silicone oil.

### (2) Pharmaceutical Composition

The pharmaceutical composition of the present invention contains the poorly water-soluble drug and the anti-aggregation agent described above in the presence of silicone oil. The phrase "in the presence of silicone oil" as used herein includes both a state in which the silicone oil is completely or partially mixed with the poorly water-soluble drug and the anti-aggregation agent and a state in which the silicone oil is not mixed with the poorly water-soluble drug and the anti-aggregation agent but are at least in contact with the poorly water-soluble drug and the anti-aggregation agent.

The silicone oil in the pharmaceutical composition of the present invention is preferably derived from the inner wall of the container, the inner wall being treated with silicone oil. The phrase "inner wall being treated with silicone oil" as used herein refers to a state in which the silicone oil is applied to the inner wall (inner surface) of the container. For example, in the case where the container is a syringe, a predetermined amount of silicone oil is applied to the inner wall of the syringe (a syringe barrel and a pusher), and the silicone oil can function as a lubricant for the pusher sliding inside the syringe barrel and a stopper (sealing plug) provided at an end portion thereof. In the case where the container is a vial, the silicone oil applied to the inner wall can reduce the likelihood that part of the content (e.g., a drug solution) filled in the vial will remain in the vial after the content is discharged to the outside. That is to say, the necessity of preliminarily setting the amount of pharmaceutical composition to be filled in the vial with consideration given to such residual content can be eliminated.

The amount of silicone oil applied to the inner wall of the container is not necessarily limited. For example, in the case where the container is a syringe, an amount of silicone oil that is sufficient to maintain the slidability of the syringe is applied to the inner wall thereof. Alternatively, for example, in the case where the container is a vial, an amount of silicone oil that enables efficient discharge of the content in the vial is applied to the inner wall thereof.

Since the pharmaceutical composition of the present invention contains the poorly water-soluble drug and the anti-aggregation agent together, even if silicone oil is applied to an inner wall of a container accommodating these ingredients and is mixed or in contact with these ingredients, aggregation of the poorly water-soluble drug when this pharmaceutical composition is suspended in an aqueous dispersion medium, which will be described later, can be prevented.

In the pharmaceutical composition of the present invention, preferably, the anti-aggregation agent is contained such that the content of the anti-aggregation ingredient is 2 to 40 parts by mass with respect to 100 parts by mass of the poorly water-soluble drug. More preferably, the anti-aggregation agent is contained such that the content of the anti-aggregation ingredient is 3 to 30 parts by mass with respect to 100 parts by mass of the poorly water-soluble drug. When the content of the anti-aggregation ingredient is 2 parts by mass or more with respect to 100 parts by mass of the poorly water-soluble drug, aggregation of the poorly water-soluble drug in an aqueous dispersion medium can be efficiently suppressed even in the presence of silicone oil. When the content of the anti-aggregation ingredient is 40 parts by mass or less with respect to 100 parts by mass of the poorly water-soluble drug, the risk that part of the poorly water-soluble drug will be dissolved due to the effect of an excessively large amount of anti-aggregation ingredient, resulting in a change in the elution property of the drug can be avoided.

Alternatively, the content of the anti-aggregation ingredient may be set based on the amount of silicone oil present in the pharmaceutical composition. In one embodiment, the content of the anti-aggregation ingredient is preferably 0.4 to 500 parts by mass, and more preferably 1.0 to 50 parts by mass, with respect to 1 part by mass of the silicone oil. When the content of the anti-aggregation ingredient is 0.4 parts by mass or more with respect to 1 part by mass of the silicone oil, aggregation of the poorly water-soluble drug in an aqueous dispersion medium can be efficiently suppressed even in the presence of the silicone oil. When the content of the anti-aggregation ingredient is 500 parts by mass or less with respect to 1 part by mass of the poorly water-soluble drug, the risk that part of the poorly water-soluble drug will be dissolved due to the effect of an excessively large amount of anti-aggregation ingredient, resulting in a change in the elution property of the drug can be avoided.

In the case where silicone oil present in the pharmaceutical composition is derived from the silicone oil applied to the inner wall of the container accommodating the pharmaceutical composition, the content of the anti-aggregation ingredient can be set by estimating the amount of silicone oil present in the pharmaceutical composition assuming that the entire amount of the silicone oil applied to the inner wall of the container is mixed into and present in the pharmaceutical composition, although, in reality, not the entire amount of the silicone oil applied to the inner wall of the container becomes mixed into the pharmaceutical composition.

Note that the amount of silicone oil present in the pharmaceutical composition can be determined by extracting the silicone oil using an appropriate solvent (e.g., methyl isobutyl ketone) and performing quantitative analysis using, for example, inductively coupled plasma (ICP) optical emission spectroscopy or atomic absorption spectrometry.

### (Other Additives)

The pharmaceutical composition of the present invention may also contain other additives, in addition to the poorly water-soluble drug and the anti-aggregation agent described above.

Examples of the other additives include, but are not necessarily limited to, other suspending agents, excipients, buffers, pH regulators, and lubricants, as well as combinations thereof.

Examples of the other suspending agents include cetylpyridinium chloride, gelatin, casein, lecithin, dextran, glycerol, acacia gum, cholesterol, tragacanth, stearic acid, benzalkonium chloride, calcium stearate, glycerol monostearate, cetostearyl alcohol, cetomacrogol emulsifying wax, sorbitan esters, polyoxyethylene castor oil derivatives, polyethylene glycols, dodecyltrimethylammonium bromide, polyoxyethylene stearate, colloidal silicon dioxide, phosphate, sodium dodecyl sulfate, carboxymethylcellulose calcium, hydroxypropyl cellulose, methylcellulose, hydroxyethyl cellulose, hydroxypropyl methylcellulose phthalate, non-crystalline cellulose, aluminum magnesium silicate, triethanolamine, polyvinyl alcohol, poloxamer, poloxamine, and charged phospholipids, as well as combinations thereof.

Examples of the excipients include mannitol, sucrose, maltose, xylitol, glucose, starch, and sorbitol, as well as combinations thereof.

Examples of the buffers include sodium phosphate, disodium hydrogen phosphate, sodium dihydrogen phosphate, potassium phosphate, and tris(hydroxymethyl)aminomethane (Tris), as well as combinations thereof.

As examples of the pH regulators, basic pH regulators can be used. For example, acidic pH regulators such as hydrochloric acid and acetic acid can be used as acidic pH regulators, and basic pH regulators such as sodium hydroxide, potassium hydroxide, calcium carbonate, magnesium oxide, and magnesium hydroxide can be used.

Examples of the lubricants include stearic acid, stearyl sodium fumarate, magnesium stearate, calcium stearate, sucrose fatty acid esters, polyethylene glycol, light anhydrous silicic acid, hydrogenated oil, glycerin fatty acid esters, and talc, as well as combinations thereof.

As for the above-listed and other additives, an appropriate amount to be added can be chosen by a person skilled in the art, as long as the effects of the present invention provided by the above-described poorly water-soluble drug and the above-described anti-aggregation agent are not impaired.

### (Shape and State)

The pharmaceutical composition of the present invention may be in any shape and any state, as long as it contains the above-described ingredients. For example, the pharmaceutical composition may be a lyophilized product in a solid state obtained by lyophilizing a suspension of the above-described ingredients dissolved or dispersed in an aqueous dispersion medium, or may be a suspension in a liquid state obtained by suspending the above-described ingredients in an aqueous dispersion medium. Preferably, the pharmaceutical composition of the present invention is obtained through lyophilization.

### (3) Medical Device

The medical device of the present invention includes a container whose inner wall is treated with silicone oil.

The container constituting the medical device of the present invention can be sealed from the outside, for example. Examples of the container include prefilled syringes and vials, preferably prefilled syringes. Specific examples of the prefilled syringes include single-chamber syringes consisting of one syringe barrel and one pusher, as well as syringes that accommodate the contents separated into a plurality of compartments (chambers) (an example of such syringes is a double-chamber syringe with two compartments).

In the case where the medical device of the present invention includes a prefilled syringe as the container, it is preferable that the prefilled syringe has the form of a double-chamber syringe that accommodates a solid containing at least a poorly water-soluble drug in one compartment and accommodates an aqueous dispersion medium in the other compartment. In this case, the above-described anti-aggregation agent, and the above-described other additives contained in the pharmaceutical composition if necessary, may be contained in the solid or in the aqueous dispersion medium.

With a medical device that has the form of a double-chamber syringe as described above, the stability of the poorly water-soluble drug can be maintained because the drug is stored as a solid until use, and great convenience can be provided when suspending the solid by adding the aqueous dispersion medium to the solid at the time of use because the aqueous dispersion medium is accommodated in the device beforehand. Furthermore, with this configuration, the coexistence of the poorly water-soluble drug and the anti-aggregation agent is kept when suspending the solid, and therefore, even when silicone oil that is applied to the inner wall of the syringe in order to increase the slidability is mixed into (present in) the suspension, the poorly water-soluble drug can be prevented from aggregating in the suspension. Thus, the dissolution profile of the poorly water-soluble drug used itself is prevented from significantly deviating from the dissolution profile of the drug before coming into contact with the silicone oil, and substantially the same sustained-release properties and efficacy can be obtained. In addition, there is thus no concern about problems such as the clogging in the device and the physical irritation to the injection site of the patient due to the increased particle size.

There is no particular limitation on the material of the container constituting the medical device of the present invention, and examples include glass, thermoplastic resins such as olefin resins (polyethylene, polypropylene, etc.) and cyclo olefin polymer (COP), and the like.

The aqueous dispersion medium is water or a mixed medium of water and ethanol. Typically, water (water for injection) is used as the aqueous dispersion medium. Note that, as described above, the aqueous dispersion medium can contain the anti-aggregation agent and the other additives (among others, other suspending agents and/or pH regulators).

### Examples

Hereinafter, the present invention will be described in greater detail using examples. However, the present invention is not limited to the examples below.

In all of the examples and comparative examples below, test suspensions were produced using a procedure that will be described below, and the volume average particle size of particles contained in the obtained suspensions was measured. In this regard, the following remarks are added in advance.

This procedure appears to be different from that which is assumed to be performed when using the medical device of the present invention and in which the poorly water-soluble drug and the anti-aggregation agent are accommodated in the container to which silicone oil is applied, and are then suspended in the aqueous dispersion medium. However, the two procedures are the same in that a poorly water-soluble drug is suspended in an aqueous dispersion medium in the presence of silicone oil. Therefore, the procedure below can be regarded as being equal to that of an example or a corresponding comparative example when a poorly water-soluble drug is suspended as assumed for the medical device of the present invention.

The volume average particle size of particles contained in the test suspensions obtained in the examples, comparative examples, and reference examples was measured in the following manner. That is, after a test suspension obtained was allowed to stand at room temperature for 5 minutes, the particle size (volume average particle size) of particles contained in this test suspension (a) was measured using a laser diffraction/scattering particle size distribution measurement apparatus (manufactured by HORIBA, Ltd. (LA-950)) (using a circulating cell and water as a measurement medium) (the results of this measurement will be referred to as "before ultrasonic treatment (a)"). Next, this test suspension (a) was ultrasonically treated for 1 minute using an ultrasonic generator attached to the particle size distribution measurement apparatus, and then, the particle size of particles contained in the resulting test suspension (b) after the ultrasonic treatment was measured in the same manner (the results of this measurement will be referred to as "after ultrasonic treatment (b)").

### (Example 1: Test Suspensions Containing Aripiprazole)

### (Example 1-1-1: Production and Evaluation of Test Suspension Using Polyoxyethylene Cetyl Ether)

An aripiprazole suspension was prepared by adding aripiprazole hydrate as a poorly water-soluble drug, carboxymethylcellulose sodium, mannitol, and sodium dihydrogen phosphate monohydrate to purified water to the following concentrations.
- Aripiprazole hydrate: 30 % by mass (as anhydride)
- Carboxymethylcellulose sodium: about 1.248 % by mass
- Mannitol: about 6.24 % by mass
- Sodium dihydrogen phosphate monohydrate: about 0.111 % by mass

To 1 g of the aripiprazole suspension was added polyoxyethylene cetyl ether as an anti-aggregation agent in an amount corresponding to 4 parts by mass with respect to 100 parts by mass of aripiprazole contained in the suspension, followed by stirring. To this suspension was added a 1% silicone oil emulsion (Dow Corning (registered trademark) 365, 35% Dimethicone NF Elusion manufactured by DuPont; hereinafter simply referred to as "1% silicone oil emulsion") such that the amount of silicone oil contained in the resulting suspension was 0.05 parts by mass with respect to 100 parts by mass of aripiprazole, followed by stirring, and thus, a test suspension (E1-1-1) was obtained. The particle size of particles contained in the obtained test suspension before ultrasonic treatment (a) and the particle size of particles contained in the test suspension after ultrasonic treatment (b) were measured. Table 1 shows the results.

### (Examples1-1-2 to 1-1-7: Production and Evaluation of Test Suspensions Using Polyoxyethylene Cetyl Ether)

To the test suspension (E1-1-1) (before ultrasonic treatment) obtained in Example 1-1-1 was added the 1% silicone oil emulsion such that the amounts of silicone oil contained in the resulting suspensions were as shown in Table 1 with respect to 100 parts by mass of aripiprazole, to thereby prepare test suspensions (E1-1-2) to (E1-1-7), and thus, the test suspensions (E1-1-2) to (E1-1-7) were obtained. The particle size of particles contained in each test suspension before ultrasonic treatment (a) and the particle size of particles contained in each test suspension after ultrasonic treatment (b) were measured. Table 1 shows the results.

### (Reference Example 1-1: Production and Evaluation of Test Suspension Using Polyoxyethylene Cetyl Ether)

A test suspension (R1-1) was prepared in the same manner as in Example 1-1-1, except that the 1% silicone oil emulsion was not added, and thus, the test suspension (R1-1) was obtained. The particle size of particles contained in the obtained test suspension before ultrasonic treatment (a) and the particle size of particles contained in the test suspension after ultrasonic treatment (b) were measured. Table 1 shows the results.

**[Table 1]**

| | | Reference Example 1-1 | Eample 1-1-1 | Example 1-1-2 | Example 1-1-3 | Example 1-1-4 | Example 1-1-5 | Example 1-1-6 | Example 1-1-7 |
|---|---|---|---|---|---|---|---|---|---|
| Ingredients (Parts by Mass) | Aripiprazole | 100 | 100 | 100 | 100 | 100 | 100 | 100 | 100 |
| | Silicone Oil | 0 | 0.05 | 0.1 | 0.2 | 0.5 | 1 | 2.5 | 5 |
| | Anti-Aggregation Agent¹⁾ | 4 | 4 | 4 | 4 | 4 | 4 | 4 | 4 |
| Amount of Anti-Aggregation Ingredient per 1 Part by Mass of Silicone Oil (Parts by Nass) | | - | 80 | 40 | 20 | 8 | 4 | 1.6 | 0.8 |
| Average Particle Size (µm) | Before Ultrasonic Treatment (a) | 4.21 | 4.47 | 4.37 | 4.51 | 4.49 | 3.59 | 4.18 | 4.39 |
| | After Ultrasonic Treatment (b) | 3.38 | 3.56 | 4.18 | 3.74 | 3.59 | 3.46 | 3.55 | 3.93 |
| | Difference ((a)-(b)) | 0.84 | 0.90 | 0.19 | 0.77 | 0.90 | 0.13 | 0.64 | 0.46 |

| | | | | | | | | | |
|---|---|---|---|---|---|---|---|---|---|
| ¹⁾ Polyoxyethylene Cetyl Ether | | | | | | | | | |

### (Example 1-2-1: Production and Evaluation of Test Suspension Using Polyoxyethylene Cetyl Ether)

A test suspension (E1-2-1) was prepared in the same manner as in Example 1-1-1, except that, to 1 g of the aripiprazole suspension prepared in Example 1-1-1, polyoxyethylene cetyl ether was added as an anti-aggregation agent in an amount corresponding to 20 parts by mass with respect to 100 parts by mass of aripiprazole contained in the suspension, and thus, the test suspension (E1-2-1) was obtained. The particle size of particles contained in the obtained test suspension before ultrasonic treatment (a) and the particle size of particles contained in the test suspension after ultrasonic treatment (b) were measured. Table 2 shows the results.

### (Examples 1-2-2 to 1-2-7: Production and Evaluation of Test Suspensions Using Polyoxyethylene Cetyl Ether)

To the test suspension (E1-2-1) (before ultrasonic treatment) obtained in Example 1-2-1 was added the 1% silicone oil emulsion such that the amounts of silicone oil contained in the resulting suspensions were as shown in Table 2 with respect to 100 parts by mass of aripiprazole, followed by stirring, and thus, test suspensions (E1-2-2) to (E1-2-7) were obtained. The particle size of particles contained in each test suspension before ultrasonic treatment (a) and the particle size of particles contained in each test suspension after ultrasonic treatment (b) were measured. Table 2 shows the results.

### (Reference Example 1-2: Production and Evaluation of Test Suspension Using Polyoxyethylene Cetyl Ether)

A test suspension (R1-2) was obtained in the same manner as in Example 1-2-1, except that the 1% silicone oil emulsion was not added. The particle size of particles contained in the obtained test suspension before ultrasonic treatment (a) and the particle size of particles contained in the test suspension after ultrasonic treatment (b) were measured. Table 2 shows the results.

**[Table 2]**

| | | Reference Example 1-2 | Example 1-2-1 | Example 1-2-2 | Example 1-2-3 | Example 1-2-4 | Example 1-2-5 | Example 1-2-6 | Example 1-2-7 |
|---|---|---|---|---|---|---|---|---|---|
| Ingredients (Parts by Mass) | Aripiprazole | 100 | 100 | 100 | 100 | 100 | 100 | 100 | 100 |
| | Silicone Oil | 0 | 0.05 | 0.1 | 0.2 | 0.5 | 1 | 2.5 | 5 |
| | Anti-Aggregation Agent¹⁾ | 20 | 20 | 20 | 20 | 20 | 20 | 20 | 20 |
| Amount of Anti-Aggregation Ingredient per 1 Part by Mass of Silicone Oil (Parts by Mass) | | - | 400 | 200 | 100 | 40 | 20 | 8 | 4 |
| Average Particle Size (µm) | Before Ultrasonic Treatment (a) | 3.54 | 3.99 | 3.47 | 3.26 | 3.31 | 3.94 | 3.97 | 3.35 |
| | After Ultrasonic Treatment (b) | 3.35 | 3.62 | 3.56 | 3.14 | 3.58 | 3.12 | 3.21 | 3.04 |
| | Difference ((a)-(b)) | 0.19 | 0.38 | -0.09 | 0.12 | -0.27 | 0.81 | 0.76 | 0.3 |

| | | | | | | | | | |
|---|---|---|---|---|---|---|---|---|---|
| ¹⁾ Polyoxyethylene Cetyl Ether | | | | | | | | | |

### (Example 1-3-1: Production and Evaluation of Test Suspension Using Polyoxyethylene (160) Polyoxypropylene (30) Glycol)

A test suspension (E1-3-1) was obtained in the same manner as in Example 1-1-1, except that, to 1 g of the aripiprazole suspension prepared in Example 1-1-1, polyoxyethylene (160) polyoxypropylene (30) glycol (having an average molecular weight of 8905) was added as an anti-aggregation agent in an amount corresponding to 4 parts by mass with respect to 100 parts by mass of aripiprazole contained in the suspension. The particle size of particles contained in the obtained test suspension before ultrasonic treatment (a) and the particle size of particles contained in the test suspension after ultrasonic treatment (b) were measured. Table 3 shows the results.

(Examples 1-3-2 to 1-3-7: Production and Evaluation of Test Suspensions Using Polyoxyethylene (160) Polyoxypropylene (30) Glycol) To the test suspension (E1-3-1) (before ultrasonic treatment) obtained in Example 1-3-1 was added the 1% silicone oil emulsion such that the amounts of silicone oil contained in the resulting suspensions were as shown in Table 3 with respect to 100 parts by mass of aripiprazole, followed by stirring, and thus, test suspensions (E1-3-2) to (E1-3-7) were obtained. The particle size of particles contained in each test suspension before ultrasonic treatment (a) and the particle size of particles contained in each test suspension after ultrasonic treatment (b) were measured. Table 3 shows the results.

### (Reference Example 1-3: Production and Evaluation of Test Suspension Using Polyoxyethylene (160) Polyoxypropylene (30) Glycol)

A test suspension (R1-3) was obtained in the same manner as in Example 1-3-1, except that the 1% silicone oil emulsion was not added. The particle size of particles contained in the obtained test suspension before ultrasonic treatment (a) and the particle size of particles contained in the test suspension after ultrasonic treatment (b) were measured. Table 3 shows the results.

**[Table 3]**

| | | Reference Example 1-3 | Example 1-3-1 | Example 1-3-2 | Example 1-3-3 | Example 1-3-4 | Example 1-3-5 | Example 1-3-6 | Example 1-3-7 |
|---|---|---|---|---|---|---|---|---|---|
| Ingredients (Parts by Mass) | Aripiprazole | 100 | 100 | 100 | 100 | 100 | 100 | 100 | 100 |
| | Silicone Oil | 0 | 0.05 | 0.1 | 0.2 | 0.5 | 1 | 2.5 | 5 |
| | Anti-Aggregation Agent¹⁾ | 4 | 4 | 4 | 4 | 4 | 4 | 4 | 4 |
| Amount of Anti-Aggregation Ingredient per 1 Part by Mass of Silicone Oil (Parts by Mass) | | - | 80 | 40 | 20 | 8 | 4 | 1.6 | 0.8 |
| Average Particle Size (µm) | Before Ultrasonic Treatment (a) | 4.62 | 4.53 | 4.22 | 4.45 | 4.10 | 5.23 | 5.74 | 5.00 |
| | After Ultrasonic Treatment (b) | 3.25 | 3.24 | 3.16 | 3.16 | 2.78 | 3.38 | 3.23 | 3.13 |
| | Difference ((a)-(b)) | 1.37 | 1.29 | 1.07 | 1.29 | 1.32 | 1.85 | 2.51 | 1.87 |

| | | | | | | | | | |
|---|---|---|---|---|---|---|---|---|---|
| ¹⁾Polyoxyethylene (160) Polyoxypropylene (30) Glycol | | | | | | | | | |

### (Example 1-4-1: Production and Evaluation of Test Suspension Using Polyoxyethylene (160) Polyoxypropylene (30) Glycol)

A test suspension (E1-4-1) was obtained in the same manner as in Example 1-1-1, except that, to 1 g of the aripiprazole suspension prepared in Example 1-1-1, polyoxyethylene (160) polyoxypropylene (30) glycol (having an average molecular weight of 8905) was added as an anti-aggregation agent in an amount corresponding to 20 parts by mass with respect to 100 parts by mass of aripiprazole contained in the suspension. The particle size of particles contained in the obtained test suspension before ultrasonic treatment (a) and the particle size of particles contained in the test suspension after ultrasonic treatment (b) were measured. Table 4 shows the results.

### (Examples 1-4-2 to 1-4-7: Production and Evaluation of Test Suspensions Using Polyoxyethylene (160) Polyoxypropylene (30) Glycol)

To the test suspension (E1-4-1) (before ultrasonic treatment) obtained in Example 1-4-1 was added the 1% silicone oil emulsion such that the amounts of silicone oil contained in the resulting suspensions were as shown in Table 4 with respect to 100 parts by mass of aripiprazole, followed by stirring, and thus, test suspensions (E1-4-2) to (E1-4-7) were obtained. The particle size of particles contained in each test suspension before ultrasonic treatment (a) and the particle size of particles contained in each test suspension after ultrasonic treatment (b) were measured. Table 4 shows the results.

### (Reference Example 1-4: Production and Evaluation of Test Suspension Using Polyoxyethylene (160) Polyoxypropylene (30) Glycol)

A test suspension (R1-4) was obtained in the same manner as in Example 1-4-1, except that the 1% silicone oil emulsion was not added. The particle size of particles contained in the obtained test suspension before ultrasonic treatment (a) and the particle size of particles contained in the test suspension after ultrasonic treatment (b) were measured. Table 4 shows the results.

**[Table 4]**

| | | Reference Example 1-4 | Example 1-4-1 | Example 1-4-2 | Example 1-4-3 | Example 1-4-4 | Example 1-4-5 | Example 1-4-6 | Example 1-4-7 |
|---|---|---|---|---|---|---|---|---|---|
| Ingredients (Parts by Mass) | Aripiprazole | 100 | 100 | 100 | 100 | 100 | 100 | 100 | 100 |
| | Silicone Oil | 0 | 0.05 | 0.1 | 0.2 | 0.5 | 1 | 2.5 | 5 |
| | Anti-Aggregation Agent¹⁾ | 20 | 20 | 20 | 20 | 20 | 20 | 20 | 20 |
| Amount of Anti-Aggregation Ingredient per 1 Part by Mass of Silicone Oil (Parts by Mass) | | - | 400 | 200 | 100 | 40 | 20 | 8 | 4 |
| Average Particle size (µm) | Before Ultrasonic Treatment (a) | 3.89 | 3.46 | 3.39 | 3.44 | 3.65 | 3.26 | 3.13 | 3.18 |
| | After Ultrasonic Treatment (b) | 3.26 | 2.85 | 2.88 | 2.94 | 3.05 | 2.80 | 2.74 | 2.61 |
| | Difference ((a)-(b)) | 0.63 | 0.61 | 0.51 | 0.50 | 0.61 | 0.46 | 0.39 | 0.57 |

| | | | | | | | | | |
|---|---|---|---|---|---|---|---|---|---|
| ¹⁾Polyoxyethylene (160) Polyoxypropylene (30) Glycol | | | | | | | | | |

### (Example 1-5-1: Production and Evaluation of Test Suspension Using Polysorbate 20)

A test suspension (E1-5-1) was obtained in the same manner as in Example 1-1-1, except that, to 1 g of the aripiprazole suspension prepared in Example 1-1-1, polysorbate 20 was added as an anti-aggregation agent in an amount corresponding to 4 parts by mass with respect to 100 parts by mass of aripiprazole contained in the suspension. The particle size of particles contained in the obtained test suspension before ultrasonic treatment (a) and the particle size of particles contained in the test suspension after ultrasonic treatment (b) were measured. Table 5 shows the results.

### (Examples 1-5-2 to 1-5-7: Production and Evaluation of Test Suspensions Using Polysorbate 20)

To the test suspension (E1-5-1) (before ultrasonic treatment) obtained in Example 1-5-1 was added the 1% silicone oil emulsion such that the amounts of silicone oil contained in the resulting suspensions were as shown in Table 5 with respect to 100 parts by mass of aripiprazole, followed by stirring, and thus, test suspensions (E1-5-2) to (E1-5-7) were obtained. The particle size of particles contained in each test suspension before ultrasonic treatment (a) and the particle size of particles contained in each test suspension after ultrasonic treatment (b) were measured. Table 5 shows the results.

### (Reference Example 1-5: Production and Evaluation of Test Suspension Using Polysorbate 20)

A test suspension (R1-5) was obtained in the same manner as in Example 1-5-1, except that the 1% silicone oil emulsion was not added. The particle size of particles contained in the obtained test suspension before ultrasonic treatment (a) and the particle size of particles contained in the test suspension after ultrasonic treatment (b) were measured. Table 5 shows the results.

**[Table 5]**

| | | Reference Example 1-5 | Example 1-5-1 | Example 1-5-2 | Example 1-5-3 | Example 1-5-4 | Example 1-5-5 | Example 1-5-6 | Example 1-5-7 |
|---|---|---|---|---|---|---|---|---|---|
| Ingredients (Parts by mass) | Aripiprazole | 100 | 100 | 100 | 100 | 100 | 100 | 100 | 100 |
| | Silicone Oil | 0 | 0.05 | 0.1 | 0.2 | 0.5 | 1 | 2.5 | 5 |
| | Anti-Aggregation Agent¹⁾ | 4 | 4 | 4 | 4 | 4 | 4 | 4 | 4 |
| Amount of Anti-Aggregation Ingredient per 1 Part by Mass of Silicone Oil (Parts by Mass) | | - | 80 | 40 | 20 | 8 | 4 | 1.6 | 0.8 |
| Average Particle Size (µm) | Before Ultrasonic Treatment (a) | 4.58 | 4.22 | 4.06 | 4.45 | 4.24 | 4.70 | 4.33 | 4.54 |
| | After Ultrasonic Treatment (b) | 3.79 | 3.74 | 3.83 | 3.72 | 3.86 | 3.97 | 3.88 | 3.26 |
| | Difference ((a)-(b)) | 0.80 | 0.48 | 0.24 | 0.73 | 0.38 | 0.73 | 0.44 | 1.28 |

| | | | | | | | | | |
|---|---|---|---|---|---|---|---|---|---|
| ¹⁾ Polysorbate 20 | | | | | | | | | |

### (Example 1-6-1: Production and Evaluation of Test Suspension Using Polysorbate 20)

A test suspension (E1-6-1) was obtained in the same manner as in Example 1-1-1, except that, to 1 g of the aripiprazole suspension prepared in Example 1-1-1, polysorbate 20 was added as an anti-aggregation agent in an amount corresponding to 20 parts by mass with respect to 100 parts by mass of aripiprazole contained in the suspension. The particle size of particles contained in the obtained test suspension before ultrasonic treatment (a) and the particle size of particles contained in the test suspension after ultrasonic treatment (b) were measured. Table 6 shows the results.

### (Examples 1-6-2 to 1-6-7: Production and Evaluation of Test Suspensions Using Polysorbate 20)

To the test suspension (E1-6-1) (before ultrasonic treatment) obtained in Example 1-6-1 was added the 1% silicone oil emulsion such that the amounts of silicone oil contained in the resulting suspensions were as shown in Table 6 with respect to 100 parts by mass of aripiprazole, followed by stirring, and thus, test suspensions (E1-6-2) to (E1-6-7) were obtained. The particle size of particles contained in each test suspension before ultrasonic treatment (a) and the particle size of particles contained in each test suspension after ultrasonic treatment (b) were measured. Table 6 shows the results.

### (Reference Example 1-6: Production and Evaluation of Test Suspension Using Polysorbate 20)

A test suspension (R1-6) was obtained in the same manner as in Example 1-6-1, except that the 1% silicone oil emulsion was not added. The particle size of particles contained in the obtained test suspension before ultrasonic treatment (a) and the particle size of particles contained in the test suspension after ultrasonic treatment (b) were measured. Table 6 shows the results.

**[Table 6]**

| | | Reference Example 1-6 | Example 1-6-1 | Example 1-6-2 | Example 1-6-3 | Example 1-6-4 | Example 1-6-5 | Example 1-6-6 | Example 1-6-7 |
|---|---|---|---|---|---|---|---|---|---|
| Ingredients (Parts by Mass) | Aripiprazole | 100 | 100 | 100 | 100 | 100 | 100 | 100 | 100 |
| | Silicone Oil | 0 | 0.05 | 0.1 | 0.2 | 0.5 | 1 | 2.5 | 5 |
| | Anti-Aggregation Aent¹⁾ | 20 | 20 | 20 | 20 | 20 | 20 | 20 | 20 |
| Amount of Anti-Aggregation Ingredient per 1 Part by Mass of Silicone oil (Parts by Mass) | | - | 400 | 200 | 100 | 40 | 20 | 8 | 4 |
| Average Particle Size (µm) | Before Ultrasonic Treatment (a) | 3.55 | 3.99 | 3.88 | 3.97 | 4.20 | 3.99 | 4.04 | 3.85 |
| | After Ultrasonic Treatment (b) | 3.41 | 3.60 | 3.66 | 3.87 | 3.61 | 3.57 | 3.68 | 3.53 |
| | Difference ((a)-(b)) | 0.15 | 0.39 | 0.22 | 0.10 | 0.59 | 0.42 | 0.36 | 0.31 |

| | | | | | | | | | |
|---|---|---|---|---|---|---|---|---|---|
| ¹⁾ Polysorbate 20 | | | | | | | | | |

### (Example 1-7-1: Production and Evaluation of Test Suspension Using Polysorbate 80)

A test suspension (E1-7-1) was obtained in the same manner as in Example 1-1-1, except that, to 1 g of the aripiprazole suspension prepared in Example 1-1-1, polysorbate 80 was added as an anti-aggregation agent in an amount corresponding to 4 parts by mass with respect to 100 parts by mass of aripiprazole contained in the suspension. The particle size of particles contained in the obtained test suspension before ultrasonic treatment (a) and the particle size of particles contained in the test suspension after ultrasonic treatment (b) were measured. Table 7 shows the results.

### (Examples 1-7-2 to 1-7-7: Production and Evaluation of Test Suspensions Using Polysorbate 80)

To the test suspension (E1-7-1) (before ultrasonic treatment) obtained in Example 1-7-1 was added the 1% silicone oil emulsion such that the amounts of silicone oil contained in the resulting suspensions were as shown in Table 7 with respect to 100 parts by mass of aripiprazole, followed by stirring, and thus, test suspensions (E1-7-2) to (E1-7-7) were obtained. The particle size of particles contained in each test suspension before ultrasonic treatment (a) and the particle size of particles contained in each test suspension after ultrasonic treatment (b) were measured. Table 7 shows the results.

### (Reference Example 1-7: Production and Evaluation of Test Suspension Using Polysorbate 80)

A test suspension (R1-7) was obtained in the same manner as in Example 1-7-1, except that the 1% silicone oil emulsion was not added. The particle size of particles contained in the obtained test suspension before ultrasonic treatment (a) and the particle size of particles contained in the test suspension after ultrasonic treatment (b) were measured. Table 7 shows the results.

**[Table 7]**

| | | Reference Example 1-7 | Example 1-7-1 | Example 1-7-2 | Example 1-7-3 | Example 1-7-4 | Example 1-7-5 | Example 1-7-6 | Example 1-7-7 |
|---|---|---|---|---|---|---|---|---|---|
| Ingredients (Parts by Mass) | Aripiprazole | 100 | 100 | 100 | 100 | 100 | 100 | 100 | 100 |
| | Silicone Oil | 0 | 0.05 | 0.1 | 0.2 | 0.5 | 1 | 2.5 | 5 |
| | Anti-Aggregation Agent¹⁾ | 4 | 4 | 4 | 4 | 4 | 4 | 4 | 4 |
| Amount of Anti-Aggregation Ingredient per 1 Part by Mass of Silicone Oil (Parts by Mass) | | - | 80 | 40 | 20 | 8 | 4 | 1.6 | 0.8 |
| Average Particle Size (µm) | Before Ultrasonic Treatment (a) | 3.91 | 4.30 | 4.35 | 4.15 | 4.38 | 4.26 | 4.18 | 4.20 |
| | After Ultrasonic Treatment (b) | 3.33 | 3.50 | 3.54 | 3.46 | 3.61 | 3.58 | 3.51 | 3.49 |
| | Difference ((a)-(b)) | 0.59 | 0.80 | 0.80 | 0.69 | 0.77 | 0.68 | 0.66 | 0.71 |

| | | | | | | | | | |
|---|---|---|---|---|---|---|---|---|---|
| ¹⁾ Polysorbate 80 | | | | | | | | | |

### (Example 1-8-1: Production and Evaluation of Test Suspension Using Polysorbate 80)

A test suspension (E1-8-1) was obtained in the same manner as in Example 1-1-1, except that, to 1 g of the aripiprazole suspension prepared in Example 1-1-1, polysorbate 80 was added as an anti-aggregation agent in an amount corresponding to 20 parts by mass with respect to 100 parts by mass of aripiprazole contained in the suspension. The particle size of particles contained in the obtained test suspension before ultrasonic treatment (a) and the particle size of particles contained in the test suspension after ultrasonic treatment (b) were measured. Table 8 shows the results.

### (Examples 1-8-2 to 1-8-7: Production and Evaluation of Test Suspensions Using Polysorbate 80)

To the test suspension (E1-8-1) (before ultrasonic treatment) obtained in Example 1-8-1 was added the 1% silicone oil emulsion such that the amounts of silicone oil contained in the resulting suspensions were as shown in Table 8 with respect to 100 parts by mass of aripiprazole, followed by stirring, and thus, test suspensions (E1-8-2) to (E1-8-7) were obtained. The particle size of particles contained in each test suspension before ultrasonic treatment (a) and the particle size of particles contained in each test suspension after ultrasonic treatment (b) were measured. Table 8 shows the results.

### (Reference Example 1-8: Production and Evaluation of Test Suspension Using Polysorbate 80)

A test suspension (R1-8) was obtained in the same manner as in Example 1-8-1, except that the 1% silicone oil emulsion was not added. The particle size of particles contained in the obtained test suspension before ultrasonic treatment (a) and the particle size of particles contained in the test suspension after ultrasonic treatment (b) were measured. Table 8 shows the results.

**[Table 8]**

| | | Reference Example 1-8 | Example 1-8-1 | Example 1-8-2 | Example 1-8-3 | Example 1-8-4 | Example 1-8-5 | Example 1-8-6 | Example 1-8-7 |
|---|---|---|---|---|---|---|---|---|---|
| Ingredients (Parts by Mass) | Aripiprazole | 100 | 100 | 100 | 100 | 100 | 100 | 100 | 100 |
| | Silicone Oil | 0 | 0.05 | 0.1 | 0.2 | 0.5 | 1 | 2.5 | 5 |
| | Anti-Aggregation Agent¹⁾ | 20 | 20 | 20 | 20 | 20 | 20 | 20 | 20 |
| Amount of Anti-Aggregation Ingredient per 1 Part by Mass of Silicone Oil (Parts by Mass) | | - | 400 | 200 | 100 | 40 | 20 | 8 | 4 |
| Average Particle Size (µm) | Before Ultrasonic Treatment (a) | 3.67 | 3.90 | 3.87 | 3.85 | 3.79 | 4.00 | 3.97 | 3.60 |
| | After Ultrasonic Treatment (b) | 3.25 | 3.95 | 3.75 | 3.74 | 3.81 | 3.96 | 3.79 | 3.38 |
| | Difference ((a)-(b)) | 0.43 | -0.05 | 0.11 | 0.11 | -0.01 | 0.04 | 0.17 | 0.22 |

| | | | | | | | | | |
|---|---|---|---|---|---|---|---|---|---|
| ¹⁾Polysorbate 80 | | | | | | | | | |

### (Comparative Example 1-1-1: Production and Evaluation of Test Suspension (Blank) Containing No Anti-Aggregation Agent)

A test suspension (C1-1-1) was obtained in the same manner as in Example 1-1-1, except that no anti-aggregation agent was added to 1 g of the aripiprazole suspension prepared in Example 1-1-1. The particle size of particles contained in the obtained test suspension before ultrasonic treatment (a) and the particle size of particles contained in the test suspension after ultrasonic treatment (b) were measured. Table 9 shows the results.

### (Comparative Examples 1-1-2 to 1-1-7: Production and Evaluation of Test Suspensions (Blanks) Containing No Anti-Aggregation Agent)

To the test suspension (C1-1-1) (before ultrasonic treatment) obtained in Comparative Example 1-1-1 was added the 1% silicone oil emulsion such that the amounts of silicone oil contained in the resulting suspensions were as shown in Table 9 with respect to 100 parts by mass of aripiprazole, followed by stirring, and thus, test suspensions (C1-1-2) to (C1-1-7) were obtained. The particle size of particles contained in each test suspension before ultrasonic treatment (a) and the particle size of particles contained in each test suspension after ultrasonic treatment (b) were measured. Table 9 shows the results.

### (Comparative Reference Example 1-1: Production and Evaluation of Test Suspension Containing No Anti-Aggregation Agent)

A test suspension (CR1-1) was obtained in the same manner as in Comparative Example 1-1-1, except that the 1% silicone oil emulsion was not added. The particle size of particles contained in the obtained test suspension before ultrasonic treatment (a) and the particle size of particles contained in the test suspension after ultrasonic treatment (b) were measured. Table 9 shows the results.

**[Table 9]**

| | | Comparative Reference Example1-1 | Comparative Example 1-1-1 | Compative Example 1-1-2 | Compative Example 1-1-3 | Compative Example 1-1-4 | Compative Example 1-1-5 | Compative Example 1-1-6 | Compative Example 1-1-7 |
|---|---|---|---|---|---|---|---|---|---|
| Ingredients (Parts by Mass) | Aripiprazole | 100 | 100 | 100 | 100 | 100 | 100 | 100 | 100 |
| | Silicone Oil | 0 | 0.05 | 0.1 | 0.2 | 0.5 | 1 | 2.5 | 5 |
| | Anti-Aggregation Agent | 0 | 0 | 0 | 0 | 0 | 0 | 0 | 0 |
| Amount of Anti-Aggregation Ingredient per 1 Part by Mass of Silicone Oil (Parts by Mass) | | - | 0 | 0 | 0 | 0 | 0 | 0 | 0 |
| Average Particle Size (µm) | Before ultrasonic treatment (a) | 3.57 | 3.52 | 3.94 | 5.44 | 8.53 | 8.10 | 13.20 | 14.49 |
| | After ultrasonic treatment (b) | 3.67 | 3.44 | 3.56 | 3.57 | 4.57 | 6.00 | 7.72 | 9.80 |
| | Difference ((a)-(b)) | -0.09 | 0.07 | 0.38 | 1.87 | 3.96 | 2.11 | 5.48 | 4.68 |

### (Reference Example 1-2-1: Production and Evaluation of Test Suspension Using Sodium Lauryl Sulfate)

A test suspension (C1-2-1) was obtained in the same manner as in Example 1-1-1, except that, to 1 g of the aripiprazole suspension prepared in Example 1-1-1, sodium lauryl sulfate was added instead of the anti-aggregation agent in an amount corresponding to 20 parts by mass with respect to 100 parts by mass of aripiprazole contained in the suspension. The particle size of particles contained in the obtained test suspension before ultrasonic treatment (a) and the particle size of particles contained in the test suspension after ultrasonic treatment (b) were measured. Table 10 shows the results.

### (Comparative Reference Example 1-2: Production and Evaluation of Test Suspension Using Sodium Lauryl Sulfate)

A test suspension (CR1-2) was obtained in the same manner as in Comparative Example 1-2-1, except that the 1% silicone oil emulsion was not added. The particle size of particles contained in the obtained test suspension before ultrasonic treatment (a) and the particle size of particles contained in the test suspension after ultrasonic treatment (b) were measured. Table 10 shows the results.

**[Table 10]**

| | | Comparative Reference Example 1-2 | Comparative Exmple 1-2-1 |
|---|---|---|---|
| Ingredients (Parts by mass) | Aripiprazole | 100 | 100 |
| | Silicone oil | 0 | 0.05 |
| | Anti-aggregation agent | 0 | 0 |
| | Sodium lauryl sulfate | 20 | 20 |
| Amount of anti-aggregation ingredient per 1 part by mass of silicone oil (Parts by mass) | | - | - |
| Average particle size (µm) | Before ultrasonic treatment (a) | 3.86 | 4.86 |
| | After ultrasonic treatment (b) | 3.77 | 5.82 |
| | Difference ((a)-(b)) | 0.10 | -0.96 |

### (Comparative Example 1-3-1: Production and Evaluation of Test Suspension Using Sodium Lauroylsarcosinate)

A test suspension (C1-3-1) was obtained in the same manner as in Example 1-1-1, except that, to 1 g of the aripiprazole suspension prepared in Example 1-1-1, sodium lauroylsarcosinate was added instead of the anti-aggregation agent in an amount corresponding to 20 parts by mass with respect to 100 parts by mass of aripiprazole contained in the suspension. The particle size of particles contained in the obtained test suspension before ultrasonic treatment (a) and the particle size of particles contained in the test suspension after ultrasonic treatment (b) were measured. Table 11 shows the results.

### (Comparative Examples 1-3-2 to 1-3-7: Production and Evaluation of Test Suspensions Using Sodium Lauroylsarcosinate)

To the test suspension (C1-3-1) (before ultrasonic treatment) obtained in Comparative Example 1-3-1 was added the 1% silicone oil emulsion such that the amounts of silicone oil contained in the resulting suspensions were as shown in Table 11 with respect to 100 parts by mass of aripiprazole, followed by stirring, and thus, test suspensions (C1-3-2) to (C1-3-7) were obtained. The particle size of particles contained in each test suspension before ultrasonic treatment (a) and the particle size of particles contained in each test suspension after ultrasonic treatment (b) were measured. Table 11 shows the results.

### (Comparative Reference Example 1-3: Production and Evaluation of Test Suspension Using Sodium Lauroylsarcosinate)

A test suspension (CR1-3) was obtained in the same manner as in Comparative Example 1-3-1, except that the 1% silicone oil emulsion was not added. The particle size of particles contained in the obtained test suspension before ultrasonic treatment (a) and the particle size of particles contained in the test suspension after ultrasonic treatment (b) were measured. Table 11 shows the results.

**[Table 11]**

| | | Comparative Reference Example 1-3 | Comparative Example 1-3-1 | Comparative Example 1-3-2 | Comparative Example 1-3-3 | Comparative Example 1-3-4 | Comparative Example 1-3-5 | Comparative Example 1-3-6 | Comparative Example 1-3-7 |
|---|---|---|---|---|---|---|---|---|---|
| Ingredients (Parts by Mass) | Aripiprazole | 100 | 100 | 100 | 100 | 100 | 100 | 100 | 100 |
| | Silicone Oil | 0 | 0.05 | 0.1 | 0.2 | 0.5 | 1 | 2.5 | 5 |
| | Anti-Aggregation Agent | 0 | 0 | 0 | 0 | 0 | 0 | 0 | 0 |
| | Sodium Lauroylsarcosinate | 20 | 20 | 20 | 20 | 20 | 20 | 20 | 20 |
| Amount of Anti-Aggregation Ingredient per 1 Part by Mass of Silicone Oil (Parts by Mass) | | - | - | - | - | - | - | - | - |
| Average Particle Size (µm) | Before Ultrasonic Treatment (a) | 4.63 | 4.35 | 5.41 | 4.19 | 4.09 | 4.46 | 4.64 | 5.99 |
| | After Ultrasonic Treatment (b) | 4.08 | 3.82 | 4.68 | 5.31 | 4.00 | 5.87 | 6.63 | 9.32 |
| | Difference ((a)-(b)) | 0.55 | 0.53 | 0.73 | -1.12 | 0.09 | -1.40 | -1.99 | -3.34 |

### (Comparative Example 1-4-1: Production and Evaluation of Test Suspension Using Polyoxyethylene (20) Polyoxypropylene (20) Glycol)

A test suspension (Cl-4-l) was obtained in the same manner as in Example 1-1-1, except that, to 1 g of the aripiprazole suspension prepared in Example 1-1-1, polyoxyethylene (20) polyoxypropylene (20) glycol (having an average molecular weight of approximately 2000 (and less than 3000)) was added instead of the anti-aggregation agent in an amount corresponding to 4 parts by mass with respect to 100 parts by mass of aripiprazole contained in the suspension, and the 1% silicone oil emulsion was not added. The particle size of particles contained in the obtained test suspension before ultrasonic treatment (a) and the particle size of particles contained in the test suspension after ultrasonic treatment (b) were measured. Table 12 shows the results. Note that the test suspension (C1-4-1) lost its fluidity and ultimately solidified.

**[Table 12]**

| | | Comparative Example 1-4-1 |
|---|---|---|
| Ingredients (Parts by Mass) | Aripiprazole | 100 |
| | Silicone Oil | 0 |
| | Anti-Aggregation Agent | 0 |
| | Polyoxyethylene (20) Polyoxypropylene (20) Glycol | 4 |
| Amount of Anti-Aggregation Ingredient per 1 Part by Mass of Silicone Oil (Parts by Mass) | | - |
| Average Particle Size (µm) | Before Ultrasonic Treatment (a) | 7.84 |
| | After Ultrasonic Treatment (b) | 3.17 |
| | Difference ((a)-(b)) | 4.67 |

### (Comparative Example 1-5-1: Production and Evaluation of Test Suspension Using Macrogol 400)

A test suspension (C1-5-1) was obtained in the same manner as in Example 1-1-1, except that, to 1 g of the aripiprazole suspension prepared in Example 1-1-1, macrogol 400 was added instead of the anti-aggregation agent in an amount corresponding to 4 parts by mass with respect to 100 parts by mass of aripiprazole contained in the suspension, and the 1% silicone oil emulsion was not added. The particle size of particles contained in the obtained test suspension before ultrasonic treatment (a) and the particle size of particles contained in the test suspension after ultrasonic treatment (b) were measured. Table 13 shows the results. Note that the test suspension (C1-5-1) lost its fluidity and ultimately solidified.

**[Table 13]**

| | | Comparative Example 1-5-1 |
|---|---|---|
| Ingredients (Parts by Mass) | Aripiprazole | 100 |
| | Silicone Oil | 0 |
| | Anti-Aggregation Agent | 0 |
| | Macrogol 400 | 4 |
| Amount of Anti-Aggregation Ingredient per 1 Part by Mass of Silicone Oil (Parts by Mass) | | - |
| Average Particle Size (µm) | Before Ultrasonic Treatment (a) | 5.74 |
| | After Ultrasonic Treatment (b) | 5.51 |
| | Difference ((a)-(b)) | 0.23 |

### (Comparative Example 1-6-1: Production and Evaluation of Test Suspension Using Macrogol 400)

A test suspension (C1-6-1) was obtained in the same manner as in Example 1-1-1, except that, to 1 g of the aripiprazole suspension prepared in Example 1-1-1, macrogol 400 was added instead of the anti-aggregation agent in an amount corresponding to 20 parts by mass with respect to 100 parts by mass of aripiprazole contained in the suspension, and the 1% silicone oil emulsion was not added. The particle size of particles contained in the obtained test suspension before ultrasonic treatment (a) and the particle size of particles contained in the test suspension after ultrasonic treatment (b) were measured. Table 14 shows the results. Note that the test suspension (C1-6-1) lost its fluidity and ultimately solidified.

**[Table 14]**

| | | Comparative Example 1-6-1 |
|---|---|---|
| Ingredients (Parts by Mass) | Aripiprazole | 100 |
| | Silicone Oil | 0 |
| | Anti-Agg regation Agent | 0 |
| | Macrogol 400 | 20 |
| Amount of Anti-Aggregation Ingredient per 1 Part by Mass of Silicone Oil (Parts by Mass) | | - |
| Average Particle Size (µm) | Before Ultrasonic Treatment (a) | 5.22 |
| | After Ultrasonic Treatment (b) | 3.11 |
| | Difference ((a)-(b)) | 2.11 |

As is clear from Tables 1 to 14 above, it can be seen that even though the test suspensions produced in the examples above contained silicone oil, aggregation of aripiprazole was appropriately prevented by the anti-aggregation agents.

### (Example 2: Test Suspensions Containing Ezetimibe)

### (Example 2-1-1: Production and Evaluation of Test Suspension Using Polyoxyethylene Cetyl Ether)

An ezetimibe suspension was prepared by adding ezetimibe as a poorly water-soluble drug, carboxymethylcellulose sodium, mannitol, and sodium dihydrogen phosphate monohydrate to purified water to the following concentrations.
- Ezetimibe: 30 % by mass
- Carboxymethylcellulose sodium: about 1.248 % by mass
- Mannitol: about 6.24 % by mass
- Sodium dihydrogen phosphate monohydrate: about 0.111 % by mass

To 1 g of the ezetimibe suspension was added polyoxyethylene cetyl ether as an anti-aggregation agent in an amount corresponding to 4 parts by mass with respect to 100 parts by mass of ezetimibe contained in the suspension, followed by stirring. To this suspension was added the 1% silicone oil emulsion such that the amount of silicone oil contained in the resulting suspension was 0.5 parts by mass with respect to 100 parts by mass of ezetimibe, followed by stirring, and thus, a test suspension (E2-1-1) was obtained. The particle size of particles contained in the obtained test suspension before ultrasonic treatment (a) and the particle size of particles contained in the test suspension after ultrasonic treatment (b) were measured. Table 15 shows the results.

### (Example 2-1-2: Production and Evaluation of Test Suspension Using Polyoxyethylene Cetyl Ether)

To the test suspension (E2-1-1) (before ultrasonic treatment) obtained in Example 2-1-1 was added the 1% silicone oil emulsion such that the amount of silicone oil contained in the resulting suspension was 5 parts by mass with respect to 100 parts by mass of ezetimibe, followed by stirring, and thus, a test suspension (E2-1-2) was obtained. The particle size of particles contained in the obtained test suspension before ultrasonic treatment (a) and the particle size of particles contained in the test suspension after ultrasonic treatment (b) were measured. Table 15 shows the results.

### (Reference Example 2-1: Production and Evaluation of Test Suspension Using Polyoxyethylene Cetyl Ether)

A test suspension (R2-1) was obtained in the same manner as in Example 2-1-1, except that the 1% silicone oil emulsion was not added. The particle size of particles contained in the obtained test suspension before ultrasonic treatment (a) and the particle size of particles contained in the test suspension after ultrasonic treatment (b) were measured. Table 15 shows the results.

**[Table 15]**

| | | Reference Example 2-1 | Example 2-1-1 | Example 2-1-2 |
|---|---|---|---|---|
| Ingredients (Parts by Mass) | Ezetimibe | 100 | 100 | 100 |
| | Silicone Oil | 0 | 0.5 | 5 |
| | Anti-Aggregation Agent¹⁾ | 4 | 4 | 4 |
| Amount of Anti-Aggregation Ingredient per 1 Part by Mass of Silicone Oil (Parts by Nass) | | - | 8 | 0.8 |
| Average Particle Size (µm) | Before Ultrasonic Treatment (a) | 3.74 | 3.63 | 3.86 |
| | After Ultrasonic Treatment (b) | 2.54 | 2.32 | 2.70 |
| | Difference ((a)-(b)) | 1.20 | 1.31 | 1.16 |

| | | | | |
|---|---|---|---|---|
| ¹⁾ Polyoxyethylene Cetyl Ether | | | | |

### (Example 2-2-1: Production and Evaluation of Test Suspension Using Polyoxyethylene (160) Polyoxypropylene (30) Glycol)

A test suspension (E2-2-1) was obtained in the same manner as in Example 2-1-1, except that, to 1 g of the ezetimibe suspension prepared in Example 2-1-1, polyoxyethylene (160) polyoxypropylene (30) glycol (having an average molecular weight of 8905) was added as an anti-aggregation agent in an amount corresponding to 4 parts by mass with respect to 100 parts by mass of ezetimibe contained in the suspension. The particle size of particles contained in the obtained test suspension before ultrasonic treatment (a) and the particle size of particles contained in the test suspension after ultrasonic treatment (b) were measured. Table 16 shows the results.

### (Example 2-2-2: Production and Evaluation of Test Suspension Using Polyoxyethylene (160) Polyoxypropylene (30) Glycol)

To the test suspension (E2-2-1) (before ultrasonic treatment) obtained in Example 2-2-1 was added the 1% silicone oil emulsion such that the amount of silicone oil contained in the resulting suspension was 5 parts by mass with respect to 100 parts by mass of ezetimibe, followed by stirring, and thus, a test suspension (E2-2-2) was obtained. The particle size of particles contained in the obtained test suspension before ultrasonic treatment (a) and the particle size of particles contained in the test suspension after ultrasonic treatment (b) were measured. Table 16 shows the results.

### (Reference Example 2-2: Production and Evaluation of Test Suspension Using Polyoxyethylene (160) Polyoxypropylene (30) Glycol)

A test suspension (R2-2) was obtained in the same manner as in Example 2-2-1, except that the 1% silicone oil emulsion was not added. The particle size of particles contained in the obtained test suspension before ultrasonic treatment (a) and the particle size of particles contained in the test suspension after ultrasonic treatment (b) were measured. Table 16 shows the results.

**[Table 16]**

| | | Reference Example 2-2 | Example 2-2-1 | Example 2-2-2 |
|---|---|---|---|---|
| Ingredients (Parts by Mass) | Ezetimibe | 100 | 100 | 100 |
| | Silicone Oil | 0 | 0.5 | 5 |
| | Anti-Aggregation Agent¹⁾ | 4 | 4 | 4 |
| Amount of Anti-Aggregation Ingredient per 1 Part by Mass of Silicone Oil (Parts by Nass) | | - | 8 | 0.8 |
| Average Particle Size (µm) | Before Ultrasonic Treatment (a) | 4.16 | 5.06 | 5.33 |
| | After Ultrasonic Treatment (b) | 2.61 | 2.62 | 2.97 |
| | Difference ((a)-(b)) | 1.55 | 2.44 | 2.36 |

| | | | | |
|---|---|---|---|---|
| ¹⁾Polyoxyethylene (160) Polyoxypropylene (30) Glycol | | | | |

(Example 2-3-1: Production and Evaluation of Test Suspension Using Polysorbate 20)

A test suspension (E2-3-1) was obtained in the same manner as in Example 2-1-1, except that, to 1 g of the ezetimibe suspension prepared in Example 2-1-1, polysorbate 20 was added as an anti-aggregation agent in an amount corresponding to 4 parts by mass with respect to 100 parts by mass of ezetimibe contained in the suspension. The particle size of particles contained in the obtained test suspension before ultrasonic treatment (a) and the particle size of particles contained in the test suspension after ultrasonic treatment (b) were measured. Table 17 shows the results.

T (Example 2-3-2: Production and Evaluation of Test Suspension Using Polysorbate 20)

o the test suspension (E2-3-1) (before ultrasonic treatment) obtained in Example 2-3-1 was added the 1% silicone oil emulsion such that the amount of silicone oil contained in the resulting suspension was 5 parts by mass with respect to 100 parts by mass of ezetimibe, followed by stirring, and thus, a test suspension (E2-3-2) was obtained. The particle size of particles contained in the obtained test suspension before ultrasonic treatment (a) and the particle size of particles contained in the test suspension after ultrasonic treatment (b) were measured. Table 17 shows the results.

### (Reference Example 2-3: Production and Evaluation of Test Suspension Using Polysorbate 20)

A test suspension (R2-3) was obtained in the same manner as in Example 2-3-1, except that the 1% silicone oil emulsion was not added. The particle size of particles contained in the obtained test suspension before ultrasonic treatment (a) and the particle size of particles contained in the test suspension after ultrasonic treatment (b) were measured. Table 17 shows the results.

**[Table 17]**

| | | Reference Example 2-3 | Example 2-3-1 | Example 2-3-2 |
|---|---|---|---|---|
| Ingredients (Parts by Mass) | Ezetimibe | 100 | 100 | 100 |
| | Silicone Oil | 0 | 0.5 | 5 |
| | Anti-Aggregation Agent¹⁾ | 4 | 4 | 4 |
| Amount of Anti-Aggregation Ingredient per 1 Part by Mass of Silicone Oil (Parts by Nass) | | - | 8 | 0.8 |
| Average Particle Size (µm) | Before Ultrasonic Treatment (a) | 5.26 | 6.96 | 10.15 |
| | After Ultrasonic Treatment (b) | 2.84 | 2.98 | 5.56 |
| | Difference ((a)-(b)) | 2.42 | 3.98 | 4.59 |

| | | | | |
|---|---|---|---|---|
| ¹⁾ Polysorbate 20 | | | | |

### (Example 2-4-1: Production and Evaluation of Test Suspension Using Polysorbate 20)

A test suspension (E2-4-1) was obtained in the same manner as in Example 2-1-1, except that, to 1 g of the ezetimibe suspension prepared in Example 2-1-1, polysorbate 20 was added as an anti-aggregation agent in an amount corresponding to 20 parts by mass with respect to 100 parts by mass of ezetimibe contained in the suspension. The particle size of particles contained in the obtained test suspension before ultrasonic treatment (a) and the particle size of particles contained in the test suspension after ultrasonic treatment (b) were measured. Table 18 shows the results.

### (Example 2-4-2: Production and Evaluation of Test Suspension Using Polysorbate 20)

To the test suspension (E2-4-1) (before ultrasonic treatment) obtained in Example 2-4-1 was added the 1% silicone oil emulsion such that the amount of silicone oil contained in the resulting suspension was 5 parts by mass with respect to 100 parts by mass of ezetimibe, followed by stirring, and thus, a test suspension (E2-4-2) was obtained. The particle size of particles contained in the obtained test suspension before ultrasonic treatment (a) and the particle size of particles contained in the test suspension after ultrasonic treatment (b) were measured. Table 18 shows the results.

### (Reference Example 2-4: Production and Evaluation of Test Suspension Using Polysorbate 20)

A test suspension (R2-4) was obtained in the same manner as in Example 2-4-1, except that the 1% silicone oil emulsion was not added. The particle size of particles contained in the obtained test suspension before ultrasonic treatment (a) and the particle size of particles contained in the test suspension after ultrasonic treatment (b) were measured. Table 18 shows the results.

**[Table 18]**

| | | Reference Example 2-4 | Example 2-4-1 | Example 2-4-2 |
|---|---|---|---|---|
| Ingredients (Parts by Mass) | Ezetimibe | 100 | 100 | 100 |
| | Silicone Oil | 0 | 0.5 | 5 |
| | Anti-Aggregation Agent¹⁾ | 20 | 20 | 20 |
| Amount of Anti-Aggregation Ingredient per 1 Part by Mass of Silicone Oil (Parts by Nass) | | - | 40 | 4 |
| Average Particle Size (µm) | Before Ultrasonic Treatment (a) | 3.55 | 5.93 | 6.96 |
| | After Ultrasonic Treatment (b) | 2.66 | 2.73 | 2.74 |
| | Difference ((a)-(b)) | 0.89 | 3.20 | 4.22 |

| | | | | |
|---|---|---|---|---|
| ¹⁾ Polysorbate 20 | | | | |

### (Example 2-5-1: Production and Evaluation of Test Suspension Using Polysorbate 80)

A test suspension (E2-5-1) was obtained in the same manner as in Example 2-1-1, except that, to 1 g of the ezetimibe suspension prepared in Example 2-1-1, polysorbate 80 was added as an anti-aggregation agent in an amount corresponding to 4 parts by mass with respect to 100 parts by mass of ezetimibe contained in the suspension. The particle size of particles contained in the obtained test suspension before ultrasonic treatment (a) and the particle size of particles contained in the test suspension after ultrasonic treatment (b) were measured. Table 19 shows the results.

### (Example 2-5-2: Production and Evaluation of Test Suspension Using Polysorbate 80)

To the test suspension (E2-5-1) (before ultrasonic treatment) obtained in Example 2-5-1 was added the 1% silicone oil emulsion such that the amount of silicone oil contained in the resulting suspension was 5 parts by mass with respect to 100 parts by mass of ezetimibe, followed by stirring, and thus, a test suspension (E2-5-2) was obtained. The particle size of particles contained in the obtained test suspension before ultrasonic treatment (a) and the particle size of particles contained in the test suspension after ultrasonic treatment (b) were measured. Table 19 shows the results.

### (Reference Example 2-5: Production and Evaluation of Test Suspension Using Polysorbate 80)

A test suspension (R2-5) was obtained in the same manner as in Example 2-5-1, except that the 1% silicone oil emulsion was not added. The particle size of particles contained in the obtained test suspension before ultrasonic treatment (a) and the particle size of particles contained in the test suspension after ultrasonic treatment (b) were measured. Table 19 shows the results.

**[Table 19]**

| | | Reference Example 2-5 | Example 2-5-1 | Example 2-5-2 |
|---|---|---|---|---|
| Ingredients (Parts by Mass) | Ezetimibe | 100 | 100 | 100 |
| | Silicone Oil | 0 | 0.5 | 5 |
| | Anti-Aggregation Agent¹⁾ | 4 | 4 | 4 |
| Amount of Anti-Aggregation Ingredient per 1 Part by Mass of Silicone Oil (Parts by Nass) | | - | 8 | 0.8 |
| Average Particle Size (µm) | Before Ultrasonic Treatment (a) | 3.65 | 4.16 | 5.32 |
| | After Ultrasonic Treatment (b) | 2.32 | 2.71 | 2.96 |
| | Difference ((a)-(b)) | 1.33 | 1.45 | 2.36 |

| | | | | |
|---|---|---|---|---|
| ¹⁾ Polysorbate 80 | | | | |

### (Example 2-6-1: Production and Evaluation of Test Suspension Using Polysorbate 80)

A test suspension (E2-6-1) was obtained in the same manner as in Example 2-1-1, except that, to 1 g of the ezetimibe suspension prepared in Example 2-1-1, polysorbate 80 was added as an anti-aggregation agent in an amount corresponding to 20 parts by mass with respect to 100 parts by mass of ezetimibe contained in the suspension. The particle size of particles contained in the obtained test suspension before ultrasonic treatment (a) and the particle size of particles contained in the test suspension after ultrasonic treatment (b) were measured. Table 20 shows the results.

### (Example 2-6-2: Production and Evaluation of Test Suspension Using Polysorbate 80)

To the test suspension (E2-6-1) (before ultrasonic treatment) obtained in Example 2-6-1 was added the 1% silicone oil emulsion such that the amount of silicone oil contained in the resulting suspension was 5 parts by mass with respect to 100 parts by mass of ezetimibe, followed by stirring, and thus, a test suspension (E2-6-2) was obtained. The particle size of particles contained in the obtained test suspension before ultrasonic treatment (a) and the particle size of particles contained in the test suspension after ultrasonic treatment (b) were measured. Table 20 shows the results.

### (Reference Example 2-6: Production and Evaluation of Test Suspension Using Polysorbate 80)

A test suspension (R2-6) was obtained in the same manner as in Example 2-6-1, except that the 1% silicone oil emulsion was not added. The particle size of particles contained in the obtained test suspension before ultrasonic treatment (a) and the particle size of particles contained in the test suspension after ultrasonic treatment (b) were measured. Table 20 shows the results.

**[Table 20]**

| | | Reference Example 2-6 | Example 2-6-1 | Example 2-6-2 |
|---|---|---|---|---|
| Ingredients (Parts by Mass) | Ezetimibe | 100 | 100 | 100 |
| | Silicone Oil | 0 | 0.5 | 5 |
| | Anti-Aggregation Agent¹) | 20 | 20 | 20 |
| Amount of Anti-Aggregation Ingredient per 1 Part by Mass of Silicone Oil (Parts by Nass) | | - | 40 | 4 |
| Average Particle Size (µm) | Before Ultrasonic Treatment (a) | 3.63 | 3.63 | 4.18 |
| | After Ultrasonic Treatment (b) | 2.48 | 2.71 | 2.71 |
| | Difference ((a)-(b)) | 1.15 | 0.92 | 1.47 |

| | | | | |
|---|---|---|---|---|
| ¹⁾ Polysorbate 80 | | | | |

### (Comparative Example 2-1-1: Production and Evaluation of Test Suspension (Blank) Containing No Anti-Aggregation Agent)

A test suspension (C2-1-1) was obtained in the same manner as in Example 2-1-1, except that no anti-aggregation agent was added to 1 g of the ezetimibe suspension prepared in Example 2-1-1. The particle size of particles contained in the obtained test suspension before ultrasonic treatment (a) and the particle size of particles contained in the test suspension after ultrasonic treatment (b) were measured. Table 21 shows the results.

### (Comparative Example 2-1-2: Production and Evaluation of Test Suspension (Blank) Containing No Anti-Aggregation Agent)

To the test suspension (C2-1-1) (before ultrasonic treatment) obtained in Comparative Example 2-1-1 was added the 1% silicone oil emulsion such that the amount of silicone oil contained in the resulting suspension was 5 parts by mass with respect to 100 parts by mass of ezetimibe, followed by stirring, and thus, a test suspension (C2-1-2) was obtained. The particle size of particles contained in the obtained test suspension before ultrasonic treatment (a) and the particle size of particles contained in the test suspension after ultrasonic treatment (b) were measured. Table 21 shows the results.

### (Comparative Reference Example 2-1: Production and Evaluation of Test Suspension Containing No Anti-Aggregation Agent)

A test suspension (CR2-1) was obtained in the same manner as in Comparative Example 2-1-1, except that the 1% silicone oil emulsion was not added. The particle size of particles contained in the obtained test suspension before ultrasonic treatment (a) and the particle size of particles contained in the test suspension after ultrasonic treatment (b) were measured. Table 21 shows the results.

**[Table 21]**

| | | Comparative Reference Example 2-1 | Comparative Example 2-1-1 | Comparative Example 2-1-2 |
|---|---|---|---|---|
| Ingredients (Parts by Mass) | Ezetimibe | 100 | 100 | 100 |
| | Silicone Oil | 0 | 0.5 | 5 |
| | Anti-Aggregation Agent | 0 | 0 | 0 |
| Amount of Anti-Aggregation Ingredient per 1 Part by Mass of Silicone Oil (Parts by Nass) | | - | 0 | 0 |
| Average Particle Size (µm) | Before Ultrasonic Treatment (a) | 5.04, | 6.42 | 17.07 |
| | After Ultrasonic Treatment (b) | 3.08 | 3.17 | 12.08 |
| | Difference ((a)-(b)) | 1.96 | 3.25 | 4.99 |

### (Comparative Example 2-2-1: Production and Evaluation of Test Suspension Using Polyoxyethylene (20) Polyoxypropylene (20) Glycol)

A test suspension (C2-2-1) was prepared in the same manner as in Example 2-1-1, except that, to 1 g of the ezetimibe suspension prepared in Example 2-1-1, polyoxyethylene (20) polyoxypropylene (20) glycol (having an average molecular weight of approximately 2000 (and less than 3000) was added instead of the anti-aggregation agent in an amount corresponding to 4 parts by mass with respect to 100 parts by mass of ezetimibe contained in the suspension. However, measurement of the particle size of particles (before ultrasonic treatment (a) and after ultrasonic treatment (b)) was not performed (Table 22), because this test suspension (C2-2-1) had already lost its fluidity and solidified, and apparent aggregation was observed visually.

### (Comparative Example 2-2-2: Production and Evaluation of Test Suspension Using Polyoxyethylene (20) Polyoxypropylene (20) Glycol)

To the test suspension (C2-2-1) (before ultrasonic treatment) obtained in Comparative Example 2-2-1 was added the 1% silicone oil emulsion such that amount of silicone oil contained in the resulting suspension was 5 parts by mass with respect to 100 parts by mass of ezetimibe, followed by stirring, and thus, a test suspension (C2-2-2) was prepared. However, measurement of the particle size of particles (before ultrasonic treatment (a) and after ultrasonic treatment (b)) was not performed (Table 22), because this test suspension (C2-2-2) had already lost its fluidity and solidified, and apparent aggregation was observed visually.

### (Comparative Reference Example 2-2: Production and Evaluation of Test Suspension Using Polyoxyethylene (20) Polyoxypropylene (20) Glycol)

A test suspension (CR2-2) was obtained in the same manner as in Comparative Example 2-2-1, except that the 1% silicone oil emulsion was not added. The particle size of particles contained in the obtained test suspension before ultrasonic treatment (a) and the particle size of particles contained in the test suspension after ultrasonic treatment (b) were measured. Table 22 shows the results. Note that the test suspension (CR2-2) lost its fluidity and was partially solidified.

**[Table 22]**

| | | Comparative Reference Example 2-2 | Comparative Example 2-2-1 | Comparative Example 2-2-2 |
|---|---|---|---|---|
| Ingredients (Parts by Mass) | Ezetimibe | 100 | 100 | 100 |
| | Silicone Oil | 0 | 0.5 | 5 |
| | Anti-Aggregation Agent | 0 | 0 | 0 |
| | Polyoxyethylene (20) Polyoxypropylene (20) Glycol | 4 | 4 | 4 |
| Amount of Anti-Aggregation Ingredient per 1 Part by Mass of Silicone Oil (Parts by Mass) | | - | - | - |
| Average Particle Size (µm) | Before Ultrasonic Treatment (a) | 10.26 | the fluidity was lost not to measured | |
| | After Ultrasonic Treatment (b) | 3.26 | | |
| | Difference ((a)-(b)) | 7.00 | | |

### (Comparative Example 2-3-1: Production and Evaluation of Test Suspension Using Macrogol 400)

A test suspension (C2-3-1) was prepared in the same manner as in Example 2-1-1, except that, to 1 g of the ezetimibe suspension prepared in Example 2-1-1, macrogol 400 was added instead of the anti-aggregation agent in an amount corresponding to 4 parts by mass with respect to 100 parts by mass of ezetimibe contained in the suspension, and the 1% silicone oil emulsion was not added. However, measurement of the particle size of particles (before ultrasonic treatment (a) and after ultrasonic treatment (b)) was not performed (Table 23), because this test suspension (C2-3-1) had already lost its fluidity and solidified, and apparent aggregation was observed visually.

### (Comparative Example 2-3-2: Production and Evaluation of Test Suspension Using Macrogol 400)

To the test suspension (C2-3-1) (before ultrasonic treatment) obtained in Comparative Example 2-3-1 was added the 1% silicone oil emulsion such that amount of silicone oil contained in the resulting suspension was 5 parts by mass with respect to 100 parts by mass of ezetimibe, followed by stirring, and thus, a test suspension (C2-3-2) was prepared. However, measurement of the particle size of particles (before ultrasonic treatment (a) and after ultrasonic treatment (b)) was not performed (Table 23), because this test suspension (C2-3-2) had already lost its fluidity and solidified, and apparent aggregation was observed visually.

### (Comparative Reference Example 2-3: Production and Evaluation of Test Suspension Using Macrogol 400)

A test suspension (CR2-3) was obtained in the same manner as in Comparative Example 2-3-1, except that the 1% silicone oil emulsion was not added. The particle size of particles contained in the obtained test suspension before ultrasonic treatment (a) and the particle size of particles contained in the test suspension after ultrasonic treatment (b) were measured. Table 23 shows the results. Note that the test suspension (CR2-3) lost its fluidity and was partially solidified.

**[Table 23]**

| | | Comparative Reference Example 2-3 | Comparative Example 2-3-1 | Comparative Example 2-3-2 |
|---|---|---|---|---|
| Ingredients (Parts by Mass) | Ezetimibe | 100 | 100 | 100 |
| | Silicone Oil | 0 | 0.5 | 5 |
| | Anti-Aggregation Agent | 0 | 0 | 0 |
| | Macrogol 400 | 4 | 4 | 4 |
| Amount of Anti-Aggregation Ingredient per 1 Part by Mass of Silicone Oil (Parts by Mass) | | - | - | - |
| Average Particle Size (µm) | Before Ultrasonic Treatment (a) | 6.54 | the fluidity was lost not to measured | |
| | After Ultrasonic Treatment (b) | 3.03 | | |
| | Difference ((a)-(b)) | 3.51 | | |

As is clear from Tables 15 to 23 above, it can be seen that even though the test suspensions produced in the examples above contained silicone oil, aggregation of ezetimibe was appropriately prevented by the anti-aggregation agents.

### (Example 3: Test Suspensions Containing Tolvaptan)

### (Example 3-1-1: Production and Evaluation of Test Suspension Using Polyoxyethylene Cetyl Ether)

A tolvaptan suspension was prepared by adding tolvaptan as a poorly water-soluble drug, carboxymethylcellulose sodium, mannitol, and sodium dihydrogen phosphate monohydrate to purified water to the following concentrations.
- Tolvaptan: 30 % by mass
- Carboxymethylcellulose sodium: about 1.248 % by mass
- Mannitol: about 6.24 % by mass
- Sodium dihydrogen phosphate monohydrate: about 0.111 % by mass

To 1 g of the tolvaptan suspension was added polyoxyethylene cetyl ether as an anti-aggregation agent in an amount corresponding to 4 parts by mass with respect to 100 parts by mass of tolvaptan contained in the suspension, followed by stirring. To this suspension was added the 1% silicone oil emulsion such that amount of silicone oil contained in the resulting suspension was 0.5 parts by mass with respect to 100 parts by mass of tolvaptan, followed by stirring, and thus, a test suspension (E3-1-1) was obtained. The particle size of particles contained in the obtained test suspension before ultrasonic treatment (a) and the particle size of particles contained in the test suspension after ultrasonic treatment (b) were measured. Table 24 shows the results.

### (Example 3-1-2: Production and Evaluation of Test Suspension Using Polyoxyethylene Cetyl Ether)

To the test suspension (E3-1-1) (before ultrasonic treatment) obtained in Example 3-1-1 was added the 1% silicone oil emulsion such that amount of silicone oil contained in the resulting suspension was 5 parts by mass with respect to 100 parts by mass of tolvaptan, followed by stirring, and thus, a test suspension (E3-1-2) was obtained. The particle size of particles contained in the obtained test suspension before ultrasonic treatment (a) and the particle size of particles contained in the test suspension after ultrasonic treatment (b) were measured. Table 24 shows the results.

### (Reference Example 3-1: Production and Evaluation of Test Suspension Using Polyoxyethylene Cetyl Ether)

A test suspension (R3-1) was obtained in the same manner as in Example 3-1-1, except that the 1% silicone oil emulsion was not added. The particle size of particles contained in the obtained test suspension before ultrasonic treatment (a) and the particle size of particles contained in the test suspension after ultrasonic treatment (b) were measured. Table 24 shows the results.

**[Table 24]**

| | | Reference Example 3-1 | Example 3-1-1 | Example 3-1-2 |
|---|---|---|---|---|
| Ingredients (Parts by mass) | Tolvaptan | 100 | 100 | 100 |
| | Silicone Oil | 0 | 0.5 | 5 |
| | Anti-Aggregation Agent¹⁾ | 4 | 4 | 4 |
| Amount of Anti-Aggregation Ingredient per 1 Part by Mass of Silicone Oil (Parts by Nass) | | - | 8 | 0.8 |
| Average Particle Size (µm) | Before Ultrasonic Treatment (a) | 14.80 | 14.38 | 13.17 |
| | After Ultrasonic Treatment (b) | 12.07 | 13.91 | 11.57 |
| | Difference ((a)-(b)) | 2.73 | 0.47 | 1.60 |

| | | | | |
|---|---|---|---|---|
| ¹⁾ Polyoxyethylene Cetyl Ether | | | | |

### (Example 3-2-1: Production and Evaluation of Test Suspension Using Polyoxyethylene (160) Polyoxypropylene (30) Glycol)

A test suspension (E3-2-1) was obtained in the same manner as in Example 3-1-1, except that, to 1 g of the tolvaptan suspension prepared in Example 3-1-1, polyoxyethylene (160) polyoxypropylene (30) glycol (having an average molecular weight of 8905) was added as an anti-aggregation agent in an amount corresponding to 4 parts by mass with respect to 100 parts by mass of tolvaptan contained in the suspension. The particle size of particles contained in the obtained test suspension before ultrasonic treatment (a) and the particle size of particles contained in the test suspension after ultrasonic treatment (b) were measured. Table 25 shows the results.

### (Example 3-2-2: Production and Evaluation of Test Suspension Using Polyoxyethylene (160) Polyoxypropylene (30) Glycol)

To the test suspension (E3-2-1) (before ultrasonic treatment) obtained in Example 3-2-1 was added the 1% silicone oil emulsion such that amount of silicone oil contained in the resulting suspension was 5 parts by mass with respect to 100 parts by mass of tolvaptan, followed by stirring, and thus, a test suspension (E3-2-2) was obtained. The particle size of particles contained in the obtained test suspension before ultrasonic treatment (a) and the particle size of particles contained in the test suspension after ultrasonic treatment (b) were measured. Table 25 shows the results.

(Reference Example 3-2: Production and Evaluation of Test Suspension Using Polyoxyethylene (160) Polyoxypropylene (30) Glycol) A test suspension (R3-2) was obtained in the same manner as in Example 3-2-1, except that the 1% silicone oil emulsion was not added. The particle size of particles contained in the obtained test suspension before ultrasonic treatment (a) and the particle size of particles contained in the test suspension after ultrasonic treatment (b) were measured. Table 25 shows the results.

**[Table 25]**

| | | Reference Example 3-2 | Example 3-2-1 | Example 3-2-2 |
|---|---|---|---|---|
| Ingredients (Parts by mass) | Tolvaptan | 100 | 100 | 100 |
| | Silicone Oil | 0 | 0.5 | 5 |
| | Anti-Aggregation Agent¹⁾ | 4 | 4 | 4 |
| Amount of Anti-Aggregation Ingredient per 1 Part by Mass of Silicone Oil (Parts by Nass) | | - | 8 | 0.8 |
| Average Particle Size (µm) | Before Ultrasonic Treatment (a) | 13.20 | 12.55 | 13.29 |
| | After Ultrasonic Treatment (b) | 12.85 | 12.34 | 13.65 |
| | Difference ((a)-(b)) | 0.35 | 0.21 | -0.36 |

| | | | | |
|---|---|---|---|---|
| ¹⁾Polyoxyethylene (160) Polyoxypropylene (30) Glycol | | | | |

### (Example 3-3-1: Production and Evaluation of Test Suspension Using Polysorbate 20)

A test suspension (E3-3-1) was obtained in the same manner as in Example 3-1-1, except that, to 1 g of the tolvaptan suspension prepared in Example 3-1-1, polysorbate 20 was added as an anti-aggregation agent in an amount corresponding to 4 parts by mass with respect to 100 parts by mass of tolvaptan contained in the suspension. The particle size of particles contained in the obtained test suspension before ultrasonic treatment (a) and the particle size of particles contained in the test suspension after ultrasonic treatment (b) were measured. Table 26 shows the results.

### (Example 3-3-2: Production and Evaluation of Test Suspension Using Polysorbate 20)

To the test suspension (E3-3-1) (before ultrasonic treatment) obtained in Example 3-3-1 was added the 1% silicone oil emulsion such that amount of silicone oil contained in the resulting suspension was 5 parts by mass with respect to 100 parts by mass of tolvaptan, followed by stirring, and thus, a test suspension (E3-3-2) was obtained. The particle size of particles contained in the obtained test suspension before ultrasonic treatment (a) and the particle size of particles contained in the test suspension after ultrasonic treatment (b) were measured. Table 26 shows the results.

### (Reference Example 3-3: Production and Evaluation of Test Suspension Using Polysorbate 20)

A test suspension (R3-3) was obtained in the same manner as in Example 3-3-1, except that the 1% silicone oil emulsion was not added. The particle size of particles contained in the obtained test suspension before ultrasonic treatment (a) and the particle size of particles contained in the test suspension after ultrasonic treatment (b) were measured. Table 26 shows the results.

**[Table 26]**

| | | Reference Example 3-3 | Example 3-3-1 | Example 3-3-2 |
|---|---|---|---|---|
| Ingredients (Parts by mass) | Tolvaptan | 100 | 100 | 100 |
| | Silicone Oil | 0 | 0.5 | 5 |
| | Anti-Aggregation Agent¹⁾ | 4 | 4 | 4 |
| Amount of Anti-Aggregation Ingredient per 1 Part by Mass of Silicone Oil (Parts by Nass) | | - | 8 | 0.8 |
| Average Particle Size (µm) | Before Ultrasonic Treatment (a) | 13.29 | 13.13 | 12.46 |
| | After Ultrasonic Treatment (b) | 12.63 | 12.13 | 11.96 |
| | Difference ((a)-(b)) | 0.66 | 1.00 | 0.50 |

| | | | | |
|---|---|---|---|---|
| ¹⁾ Polysorbate 20 | | | | |

### (Example 3-4-1: Production and Evaluation of Test Suspension Using Polysorbate 80)

A test suspension (E3-4-1) was obtained in the same manner as in Example 3-1-1, except that, to 1 g of the tolvaptan suspension prepared in Example 3-1-1, polysorbate 80 was added as an anti-aggregation agent in an amount corresponding to 4 parts by mass with respect to 100 parts by mass of tolvaptan contained in the suspension. The particle size of particles contained in the obtained test suspension before ultrasonic treatment (a) and the particle size of particles contained in the test suspension after ultrasonic treatment (b) were measured. Table 27 shows the results.

### (Example 3-4-2: Production and Evaluation of Test Suspension Using Polysorbate 80)

To the test suspension (E3-4-1) (before ultrasonic treatment) obtained in Example 3-4-1 was added the 1% silicone oil emulsion such that amount of silicone oil contained in the resulting suspension was 5 parts by mass with respect to 100 parts by mass of tolvaptan, followed by stirring, and thus, a test suspension (E3-4-2) was obtained. The particle size of particles contained in the obtained test suspension before ultrasonic treatment (a) and the particle size of particles contained in the test suspension after ultrasonic treatment (b) were measured. Table 27 shows the results.

### (Reference Example 3-4: Production and Evaluation of Test Suspension Using Polysorbate 80)

A test suspension (R3-4) was obtained in the same manner as in Example 3-4-1, except that the 1% silicone oil emulsion was not added. The particle size of particles contained in the obtained test suspension before ultrasonic treatment (a) and the particle size of particles contained in the test suspension after ultrasonic treatment (b) were measured. Table 27 shows the results.

**[Table 27]**

| | | Reference Example 3-4 | Example 3-4-1 | Example 3-4-2 |
|---|---|---|---|---|
| Ingredients (Parts by mass) | Tolvaptan | 100 | 100 | 100 |
| | Silicone Oil | 0 | 0.5 | 5 |
| | Anti-Aggregation Agent¹⁾ | 4 | 4 | 4 |
| Amount of Anti-Aggregation Ingredient per 1 Part by Mass of Silicone Oil (Parts by Nass) | | - | 8 | 0.8 |
| Average Particle Size (µm) | Before Ultrasonic Treatment (a) | 10.83 | 12.26 | 10.17 |
| | After Ultrasonic Treatment (b) | 9.02 | 11.83 | 8.49 |
| | Difference ((a)-(b)) | 1.81 | 0.43 | 1.68 |

| | | | | |
|---|---|---|---|---|
| ¹⁾ Polysorbate 80 | | | | |

### (Comparative Example 3-1-1: Production and Evaluation of Test Suspension (Blank) Containing No Anti-Aggregation Agent)

A test suspension (C3-1-1) was obtained in the same manner as in Example 3-1-1, except that no anti-aggregation agent was added to 1 g of the tolvaptan suspension prepared in Example 3-1-1. The particle size of particles contained in the obtained test suspension before ultrasonic treatment (a) and the particle size of particles contained in the test suspension after ultrasonic treatment (b) were measured. Table 28 shows the results.

### (Comparative Example 3-1-2: Production and Evaluation of Test Suspension (Blank) Containing No Anti-Aggregation Agent)

To the test suspension (C3-1-1) (before ultrasonic treatment) obtained in Comparative Example 3-1-1 was added the 1% silicone oil emulsion such that amount of silicone oil contained in the resulting suspension was 5 parts by mass with respect to 100 parts by mass of tolvaptan, followed by stirring, and thus, a test suspension (C3-1-2) was obtained. The particle size of particles contained in the obtained test suspension before ultrasonic treatment (a) and the particle size of particles contained in the test suspension after ultrasonic treatment (b) were measured. Table 28 shows the results.

### (Comparative Reference Example 3-1: Production and Evaluation of Test Suspension Containing No Anti-Aggregation Agent)

A test suspension (CR3-1) was obtained in the same manner as in Comparative Example 3-1-1, except that the 1% silicone oil emulsion was not added. The particle size of particles contained in the obtained test suspension before ultrasonic treatment (a) and the particle size of particles contained in the test suspension after ultrasonic treatment (b) were measured. Table 28 shows the results.

**[Table 28]**

| | | Comparative Reference Example 3-1 | Comparative Example 3-1-1 | Comparative Example 3-1-2 |
|---|---|---|---|---|
| Ingredients (Parts by mass) | Tolvaptan | 100 | 100 | 100 |
| | Silicone Oil | 0 | 0.5 | 5 |
| | Anti-Aggregation Agent | 0 | 0 | 0 |
| Amount of Anti-Aggregation Ingredient per 1 Part by Mass of Silicone Oil (Parts by Nass) | | - | 0 | 0 |
| Average Particle Size (µm) | Before Ultrasonic Treatment (a) | 11.35 | 14.80 | 25.66 |
| | After Ultrasonic Treatment (b) | 9.98 | 12.39 | 18.99 |
| | Difference ((a)-(b)) | 1.37 | 2.41 | 6.67 |

As is clear from Tables 24 to 28 above, it can be seen that even though the test suspensions produced in the examples above contained silicone oil, aggregation of tolvaptan was appropriately prevented by the anti-aggregation agents.

### (Example 4: Test Suspensions Containing Sorafenib Tosylate) (Example 4-1-1: Production and Evaluation of Test Suspension Using Polyoxyethylene Cetyl Ether)

A sorafenib tosylate suspension was prepared by adding sorafenib tosylate as a poorly water-soluble drug, carboxymethylcellulose sodium, mannitol, and sodium dihydrogen phosphate monohydrate to purified water to the following concentrations.
- Sorafenib tosylate: 30 % by mass
- Carboxymethylcellulose sodium: about 1.248 % by mass
- Mannitol: about 6.24 % by mass
- Sodium dihydrogen phosphate monohydrate: about 0.111 % by mass

To 1 g of the sorafenib tosylate suspension was added polyoxyethylene cetyl ether as an anti-aggregation agent in an amount corresponding to 4 parts by mass with respect to 100 parts by mass of sorafenib tosylate contained in the suspension, followed by stirring. To this suspension was added the 1% silicone oil emulsion such that the amount of silicone oil contained in the resulting suspension was 0.5 parts by mass with respect to 100 parts by mass of sorafenib tosylate, followed by stirring, and thus, a test suspension (E4-1-1) was obtained. The particle size of particles contained in the obtained test suspension before ultrasonic treatment (a) and the particle size of particles contained in the test suspension after ultrasonic treatment (b) were measured. Table 29 shows the results.

### (Example 4-1-2: Production and Evaluation of Test Suspension Using Polyoxyethylene Cetyl Ether)

To the test suspension (E4-1-1) (before ultrasonic treatment) obtained in Example 4-1-1 was added the 1% silicone oil emulsion such that the amount of silicone oil contained in the resulting suspension was 5 parts by mass with respect to 100 parts by mass of sorafenib tosylate, followed by stirring, and thus, a test suspension (E4-1-2) was obtained. The particle size of particles contained in the obtained test suspension before ultrasonic treatment (a) and the particle size of particles contained in the test suspension after ultrasonic treatment (b) were measured. Table 29 shows the results.

### (Reference Example 4-1: Production and Evaluation of Test Suspension Using Polyoxyethylene Cetyl Ether)

A test suspension (R4-1) was obtained in the same manner as in Example 4-1-1, except that the 1% silicone oil emulsion was not added. The particle size of particles contained in the obtained test suspension before ultrasonic treatment (a) and the particle size of particles contained in the test suspension after ultrasonic treatment (b) were measured. Table 29 shows the results.

**[Table 29]**

| | | Reference Example 4-1 | Example 4-1-1 | Example 4-1-2 |
|---|---|---|---|---|
| Ingredients (Parts by Mass) | Sorafenib Tosylate | 100 | 100 | 100 |
| | Silicone Oil | 0 | 0.5 | 5 |
| | Anti-Aggregation Agent¹⁾ | 4 | 4 | 4 |
| Amount of Anti-Aggregation Ingredient per 1 Part by Mass of Silicone Oil (Parts by Nass) | | - | 8 | 0.8 |
| Average Particle Size (µm) | Before Ultrasonic Treatment (a) | 39.16 | 40.84 | 40.26 |
| | After Ultrasonic Treatment (b) | 30.98 | 31.60 | 26.51 |
| | Difference ((a)-(b)) | 8.18 | 9.24 | 13.75 |

| | | | | |
|---|---|---|---|---|
| ¹⁾ Polyoxyethylene Cetyl Ether | | | | |

### (Example 4-2-1: Production and Evaluation of Test Suspension Using Polyoxyethylene (160) Polyoxypropylene (30) Glycol)

A test suspension (E4-2-1) was obtained in the same manner as in Example 4-1-1, except that, to 1 g of the sorafenib tosylate suspension prepared in Example 4-1-1, polyoxyethylene (160) polyoxypropylene (30) glycol (having an average molecular weight of 8905) was added as an anti-aggregation agent in an amount corresponding to 4 parts by mass with respect to 100 parts by mass of sorafenib tosylate contained in the suspension. The particle size of particles contained in the obtained test suspension before ultrasonic treatment (a) and the particle size of particles contained in the test suspension after ultrasonic treatment (b) were measured. Table 30 shows the results.

### (Example 4-2-2: Production and Evaluation of Test Suspension Using Polyoxyethylene (160) Polyoxypropylene (30) Glycol)

To the test suspension (E4-2-1) (before ultrasonic treatment) obtained in Example 4-2-1 was added the 1% silicone oil emulsion such that the amount of silicone oil contained in the resulting suspension was 5 parts by mass with respect to 100 parts by mass of sorafenib tosylate, followed by stirring, and thus, a test suspension (E4-2-2) was obtained. The particle size of particles contained in the obtained test suspension before ultrasonic treatment (a) and the particle size of particles contained in the test suspension after ultrasonic treatment (b) were measured. Table 30 shows the results.

### (Reference Example 4-2: Production and Evaluation of Test Suspension Using Polyoxyethylene (160) Polyoxypropylene (30) Glycol)

A test suspension (R4-2) was obtained in the same manner as in Example 4-2-1, except that the 1% silicone oil emulsion was not added. The particle size of particles contained in the obtained test suspension before ultrasonic treatment (a) and the particle size of particles contained in the test suspension after ultrasonic treatment (b) were measured. Table 30 shows the results.

**[Table 30]**

| | | Reference Example 4-2 | Example 4-2-1 | Example 4-2-2 |
|---|---|---|---|---|
| Ingredients (Parts by Mass) | Sorafenib Tosylate | 100 | 100 | 100 |
| | Silicone Oil | 0 | 0.5 | 5 |
| | Anti-Aggregation Agent¹⁾ | 4 | 4 | 4 |
| Amount of Anti-Aggregation Ingredient per 1 Part by Mass of Silicone Oil (Parts by Nass) | | - | 8 | 0.8 |
| Average Particle Size (µm) | Before Ultrasonic Treatment (a) | 38.03 | 37.68 | 37.83 |
| | After Ultrasonic Treatment (b) | 30.23 | 27.79 | 26.25 |
| | Difference ((a)-(b)) | 7.80 | 9.89 | 11.58 |

| | | | | |
|---|---|---|---|---|
| ¹⁾Polyoxyethylene (160) Polyoxypropylene (30) Glycol | | | | |

### (Example 4-3-1: Production and Evaluation of Test Suspension Using Polysorbate 20)

A test suspension (E4-3-1) was obtained in the same manner as in Example 4-1-1, except that, to 1 g of the sorafenib tosylate suspension prepared in Example 4-1-1, polysorbate 20 was added as an anti-aggregation agent in an amount corresponding to 4 parts by mass with respect to 100 parts by mass of sorafenib tosylate contained in the suspension. The particle size of particles contained in the obtained test suspension before ultrasonic treatment (a) and the particle size of particles contained in the test suspension after ultrasonic treatment (b) were measured. Table 31 shows the results.

### (Example 4-3-2: Production and Evaluation of Test Suspension Using Polysorbate 20)

To the test suspension (E4-3-1) (before ultrasonic treatment) obtained in Example 4-3-1 was added the 1% silicone oil emulsion such that the amount of silicone oil contained in the resulting suspension was 5 parts by mass with respect to 100 parts by mass of sorafenib tosylate, followed by stirring, and thus, a test suspension (E4-3-2) was obtained. The particle size of particles contained in the obtained test suspension before ultrasonic treatment (a) and the particle size of particles contained in the test suspension after ultrasonic treatment (b) were measured. Table 31 shows the results.

### (Reference Example 4-3: Production and Evaluation of Test Suspension Using Polysorbate 20)

A test suspension (R4-3) was obtained in the same manner as in Example 4-3-1, except that the 1% silicone oil emulsion was not added. The particle size of particles contained in the obtained test suspension before ultrasonic treatment (a) and the particle size of particles contained in the test suspension after ultrasonic treatment (b) were measured. Table 31 shows the results.

**[Table 31]**

| | | Reference Example 4-3 | Example 4-3-1 | Example 4-3-2 |
|---|---|---|---|---|
| Ingredients (Parts by Mass) | Sorafenib Tosylate | 100 | 100 | 100 |
| | Silicone Oil | 0 | 0.5 | 5 |
| | Anti-Aggregation Agent¹⁾ | 4 | 4 | 4 |
| Amount of Anti-Aggregation Ingredient per 1 Part by Mass of Silicone Oil (Parts by Nass) | | - | 8 | 0.8 |
| Average Particle Size (µm) | Before Ultrasonic Treatment (a) | 40.11 | 38.48 | 40.39 |
| | After Ultrasonic Treatment (b) | 26.62 | 27.34 | 26.39 |
| | Difference ((a)-(b)) | 13.49 | 11.14 | 14.00 |

| | | | | |
|---|---|---|---|---|
| ¹⁾ Polysorbate 20 | | | | |

### (Example 4-4-1: Production and Evaluation of Test Suspension Using Polysorbate 80)

A test suspension (E4-4-1) was obtained in the same manner as in Example 4-1-1, except that, to 1 g of the sorafenib tosylate suspension prepared in Example 4-1-1, polysorbate 80 was added as an anti-aggregation agent in an amount corresponding to 4 parts by mass with respect to 100 parts by mass of sorafenib tosylate contained in the suspension. The particle size of particles contained in the obtained test suspension before ultrasonic treatment (a) and the particle size of particles contained in the test suspension after ultrasonic treatment (b) were measured. Table 32 shows the results.

### (Example 4-4-2: Production and Evaluation of Test Suspension Using Polysorbate 80)

To the test suspension (E4-4-1) (before ultrasonic treatment) obtained in Example 4-4-1 was added the 1% silicone oil emulsion such that the amount of silicone oil contained in the resulting suspension was 5 parts by mass with respect to 100 parts by mass of sorafenib tosylate, followed by stirring, and thus, a test suspension (E4-4-2) was obtained. The particle size of particles contained in the obtained test suspension before ultrasonic treatment (a) and the particle size of particles contained in the test suspension after ultrasonic treatment (b) were measured. Table 32 shows the results.

### (Reference Example 4-4: Production and Evaluation of Test Suspension Using Polysorbate 80)

A test suspension (R4-4) was obtained in the same manner as in Example 4-4-1, except that the 1% silicone oil emulsion was not added. The particle size of particles contained in the obtained test suspension before ultrasonic treatment (a) and the particle size of particles contained in the test suspension after ultrasonic treatment (b) were measured. Table 32 shows the results.

**[Table 32]**

| | | Reference Example 4-4 | Example 4-4-1 | Example 4-4-2 |
|---|---|---|---|---|
| Ingredients (Parts by Mass) | Sorafenib Tosylate | 100 | 100 | 100 |
| | Silicone Oil | 0 | 0.5 | 5 |
| | Anti-Aggregation Agent¹⁾ | 4 | 4 | 4 |
| Amount of Anti-Aggregation Ingredient per 1 Part by Mass of Silicone Oil (Parts by Nass) | | - | 8 | 0.8 |
| Average Particle Size (µm) | Before Ultrasonic Treatment (a) | 39.20 | 40.19 | 39.38 |
| | After Ultrasonic Treatment (b) | 25.13 | 26.87 | 27.06 |
| | Difference ((a)-(b)) | 14.07 | 13.32 | 12.32 |

| | | | | |
|---|---|---|---|---|
| ¹⁾ Polysorbate 80 | | | | |

### (Comparative Example 4-1-1: Production and Evaluation of Test Suspension (Blank) Containing No Anti-Aggregation Agent)

A test suspension (C4-1-1) was obtained in the same manner as in Example 4-1-1, except that no anti-aggregation agent was added to 1 g of the sorafenib tosylate suspension prepared in Example 4-1-1. The particle size of particles contained in the obtained test suspension before ultrasonic treatment (a) and the particle size of particles contained in the test suspension after ultrasonic treatment (b) were measured. Table 33 shows the results.

### (Comparative Example 4-1-2: Production and Evaluation of Test Suspension (Blank) Containing No Anti-Aggregation Agent)

To the test suspension (C4-1-1) (before ultrasonic treatment) obtained in Comparative Example 4-1-1 was added the 1% silicone oil emulsion such that the amount of silicone oil contained in the resulting suspension was 5 parts by mass with respect to 100 parts by mass of sorafenib tosylate, followed by stirring, and thus, a test suspension (C4-1-2) was obtained. The particle size of particles contained in the obtained test suspension before ultrasonic treatment (a) and the particle size of particles contained in the test suspension after ultrasonic treatment (b) were measured. Table 33 shows the results.

### (Comparative Reference Example 4-1: Production and Evaluation of Test Suspension Containing No Anti-Aggregation Agent)

A test suspension (CR4-1) was obtained in the same manner as in Comparative Example 4-1-1, except that the 1% silicone oil emulsion was not added. The particle size of particles contained in the obtained test suspension before ultrasonic treatment (a) and the particle size of particles contained in the test suspension after ultrasonic treatment (b) were measured. Table 33 shows the results.

**[Table 33]**

| | | Comparative Reference Example 4-1 | Comparative Example 4-1-1 | Comparative Example 4-1-2 |
|---|---|---|---|---|
| Ingredients (Parts by Mass) | Sorafenib Tosylate | 100 | 100 | 100 |
| | Silicone Oil | 0 | 0.5 | 5 |
| | Anti-Aggregation Agent | 0 | 0 | 0 |
| Amount of Anti-Aggregation Ingredient per 1 Part by Mass of Silicone Oil (Parts by Nass) | | - | 0 | 0 |
| Average Particle Size (µm) | Before Ultrasonic Treatment (a) | 37.80 | 46.81 | 52.91 |
| | After Ultrasonic Treatment (b) | 24.22 | 29.68 | 31.58 |
| | Difference ((a)-(b)) | 13.58 | 17.13 | 21.33 |

### (Comparative Example 4-2-1: Production and Evaluation of Test Suspension Using Sodium Lauroylsarcosinate)

A test suspension (C4-2-1) was obtained in the same manner as in Example 4-1-1, except that, to 1 g of the tolvaptan suspension prepared in Example 4-1-1, sodium lauroylsarcosinate was added instead of the anti-aggregation agent in an amount corresponding to 4 parts by mass with respect to 100 parts by mass of sorafenib tosylate contained in the suspension. The particle size of particles contained in the obtained test suspension before ultrasonic treatment (a) and the particle size of particles contained in the test suspension after ultrasonic treatment (b) were measured. Table 34 shows the results.

### (Comparative Example 4-2-2: Production and Evaluation of Test Suspension Using Sodium Lauroylsarcosinate)

To the test suspension (C4-2-1) (before ultrasonic treatment) obtained in Comparative Example 4-2-1 was added the 1% silicone oil emulsion such that the amount of silicone oil contained in the resulting suspension was 5 parts by mass with respect to 100 parts by mass of sorafenib tosylate, followed by stirring, and thus, a test suspension (C4-2-2) was obtained. The particle size of particles contained in the obtained test suspension before ultrasonic treatment (a) and the particle size of particles contained in the test suspension after ultrasonic treatment (b) were measured. Table 34 shows the results.

### (Comparative Reference Example 4-2: Production and Evaluation of Test Suspension Using Sodium Lauroylsarcosinate)

A test suspension (CR4-2) was obtained in the same manner as in Comparative Example 4-2-1, except that the 1% silicone oil emulsion was not added. The particle size of particles contained in the obtained test suspension before ultrasonic treatment (a) and the particle size of particles contained in the test suspension after ultrasonic treatment (b) were measured. Table 34 shows the results. Note that the test suspension (CR4-2) lost its fluidity and was partially solidified.

**[Table 34]**

| | | Comparative Reference Example 4-2 | Comparative Example 4-2-1 | Comparative Example 4-2-2 |
|---|---|---|---|---|
| Ingredients (Parts by Mass) | Sorafenib Tosylate | 100 | 100 | 100 |
| | Silicone Oil | 0 | 0.5 | 5 |
| | Anti-Aggregation Agent | 0 | 0 | 0 |
| | Sodium Lauroylsarcosinate | 4 | 4 | 4 |
| Amount of Anti-Aggregation Ingredient per 1 Part by Mass of Silicone Oil (Parts by Nass) | | - | - | - |
| Average Particle Size (µm) | Before Ultrasonic Treatment (a) | 49.78 | 76.58 | 71.19 |
| | After Ultrasonic Treatment (b) | 32.49 | 34.71 | 35.00 |
| | Difference ((a)-(b)) | 17.29 | 41.87 | 36.19 |

### (Comparative Example 4-3-1: Production and Evaluation of Test Suspension Using Polyoxyethylene (20) Polyoxypropylene (20) Glycol)

A test suspension (C4-3-1) was obtained in the same manner as in Example 4-1-1, except that, to 1 g of the tolvaptan suspension prepared in Example 4-1-1, polyoxyethylene (20) polyoxypropylene (20) glycol (having an average molecular weight of approximately 2000 (and less than 3000)) was added instead of the anti-aggregation agent in an amount corresponding to 4 parts by mass with respect to 100 parts by mass of sorafenib tosylate contained in the suspension. The particle size of particles contained in the obtained test suspension before ultrasonic treatment (a) and the particle size of particles contained in the test suspension after ultrasonic treatment (b) were measured. Table 35 shows the results.

### (Comparative Example 4-3-2: Production and Evaluation of Test Suspension Using Polyoxyethylene (20) Polyoxypropylene (20) Glycol)

To the test suspension (C4-3-1) (before ultrasonic treatment) obtained in Comparative Example 4-3-1 was added the 1% silicone oil emulsion such that amount of the silicone oil contained in the resulting suspension was 5 parts by mass with respect to 100 parts by mass of sorafenib tosylate, followed by stirring, and thus, a test suspension (C4-3-2) was obtained. The particle size of particles contained in the obtained test suspension before ultrasonic treatment (a) and the particle size of particles contained in the test suspension after ultrasonic treatment (b) were measured. Table 35 shows the results.

### (Comparative Reference Example 4-3: Production and Evaluation of Test Suspension Using Polyoxyethylene (20) Polyoxypropylene (20) Glycol)

A test suspension (CR4-3) was obtained in the same manner as in Comparative Example 4-3-1, except that the 1% silicone oil emulsion was not added. The particle size of particles contained in the obtained test suspension before ultrasonic treatment (a) and the particle size of particles contained in the test suspension after ultrasonic treatment (b) were measured. Table 35 shows the results. Note that the test suspension (CR4-3) lost its fluidity and was partially solidified.

**[Table 35]**

| | | Comparative Reference Example 4-3 | Comparative Example 4-3-1 | Comparative Example 4-3-2 |
|---|---|---|---|---|
| Ingredients (Parts by Mass) | Sorafenib Tosylate | 100 | 100 | 100 |
| | Silicone Oil | 0 | 0.5 | 5 |
| | Anti-Aggregation Agent | 0 | 0 | 0 |
| | Polyoxyethylene (20) Polyoxypropylene (20) Glycol | 4 | 4 | 4 |
| Amount of Anti-Aggregation Ingredient per 1 Part by Mass of Silicone Oil (Parts by Mass) | | - | - | - |
| Average Particle Size (µm) | Before Ultrasonic Treatment (a) | 49.47 | 53.99 | 58.12 |
| | After Ultrasonic Treatment (b) | 32.61 | 35.24 | 30.39 |
| | Difference ((a)-(b)) | 16.86 | 18.75 | 27.73 |

As is clear from Tables 29 to 35 above, it can be seen that even though the test suspensions produced in the examples above contained silicone oil, aggregation of sorafenib tosylate was appropriately prevented by the anti-aggregation agents.

Thus, it can be seen that the anti-aggregation agents used in the examples above are useful for preventing poorly water-soluble drugs, such as aripiprazole, ezetimibe, tolvaptan, and sorafenib tosylate, from aggregating when suspended in an aqueous dispersion medium in the presence of silicone oil. Furthermore, it can also be seen that the coexistence of such an anti-aggregation agent with a poorly water-soluble drug in a medical device including a container whose inner wall is treated with silicone oil is useful.

### Industrial Applicability

The present invention is useful in the fields of manufacturing pharmaceuticals and medical devices, for example.

## Claims

1. An anti-aggregation agent for preventing aggregation of a poorly water-soluble drug caused by silicone oil,
comprising, as an anti-aggregation ingredient, at least one selected from the group consisting of polyoxyethylene cetyl ether, polyoxyethylene sorbitan fatty acid esters, and polyoxyethylene polyoxypropylene glycols having an average molecular weight of 3000 to 13000.

2. A pharmaceutical composition comprising a poorly water-soluble drug and the anti-aggregation agent according to claim 1 in the presence of silicone oil.

3. The pharmaceutical composition according to claim 2, wherein the anti-aggregation agent contains the anti-aggregation ingredient in an amount of 2 to 40 parts by mass with respect to 100 parts by mass of the drug.

4. The pharmaceutical composition according to claim 2 or 3, wherein the silicone oil is derived from an inner wall of a container, and the inner wall being treated with silicone oil.

5. The pharmaceutical composition according to any one of claims 2 to 4, which is obtained through lyophilization.

6. A medical device comprising a container whose inner wall is treated with silicone oil,
wherein a poorly water-soluble drug and the anti-aggregation agent according to claim 1 are accommodated in the container.

7. The medical device according to claim 6, which has a form of a prefilled syringe.

8. The medical device according to claim 6 or 7, wherein the anti-aggregation agent contains the anti-aggregation ingredient in an amount of 2 to 40 parts by mass with respect to 100 parts by mass of the drug.
